# EUROPÄISCHE PATENTANMELDUNG

(11) **EP 2 280 076 A1**
(43) Veröffentlichungstag der Anmeldung: **02.02.2011**
(21) Anmeldenummer: 10007349.3
(22) Anmeldetag: 15.07.2010
(51) Int. Cl.: C12P 21/02, C12N 9/08, C12N 15/80, C12N 15/53

(54) **Verfahren zur Expression von homologen Proteinen und/oder Peptiden in Pilzen der Klasse Dothideomycetes**

(30) Priorität: 30.07.2009 DE 102009035671
(71) Anmelder: DECHEMA Gesellschaft für Chemische Technik und Biotechnologie e.V., 60486 Frankfurt (DE)
(72) Erfinder: Buchhaupt, Markus, Dr., 61138 Niederdorfelden (DE); Schrader, Jens, Dr., 60320 Frankfurt am Main (DE); Guder, Jan Christopher, 67269 Grünstadt (DE); Ehrich, Kristina, 65933 Frankfurt am Main (DE)
(74) Vertreter: Köllner, Malte

(57) **Zusammenfassung**

Die vorliegende Erfindung betrifft ein Verfahren zur Expression von Proteinen und Peptiden im Pilz *Caldariomyces* fumago und anderen Vertretern der Klasse *Dothideomycetes*. Dabei wird eine Expressionskassette, welche mindestens eine Gensequenz für ein homologes Protein oder Peptid und einen Selektionsmarker enthält, in die ribosomale DNA, bevorzugt in die nicht transkribierten Bereiche der ribosomalen DNA des Zielorganismus integriert.

## Beschreibung

### Gebiet der Erfindung

Die vorliegende Erfindung betrifft ein Verfahren zur Expression von homologen Proteinen und/oder Peptiden im Pilz *Caldariomyces fumago* und anderen Vertretern der Klasse *Dothideomycetes.*

### Stand der Technik

Die Expression von Proteinen und Peptiden in Organismen gehört zu einer der wichtigsten Anwendungen der modernen Biotechnologie. Allerdings ist es häufig nicht möglich, alle Proteine und Peptide in ein und demselben Organismus zu exprimieren. Vielmehr bedingen insbesondere posttranslationale Modifikationen, dass manche Proteine nur von bestimmten Organismen hergestellt werden können. Es ist daher von Vorteil die Proteine im gleichen Organismus zu exprimieren, aus dem sie stammen (homologe Proteine). Allerdings sind gerade diese oft nur in geringer Menge vorhanden.

Gerade Pilze weisen oft eine besondere Art von Enzymen auf, welche sich durch besondere Reaktionen und Eigenschaften auszeichnen. Dies wird zum Beispiel an der Chloroperoxidase (CPO) aus *Caldariomyces fumago* (*C. fumago*) deutlich. Dieses Enzym ist vielfach posttranslational modifiziert, beispielsweise durch Glykosidierungen. Eine heterologe Überexpression des CPO-Gens in anderen Organismen wie *Escherichia coli*, *Pichia pastoris*, Sf9-Insektenzellen (Zong 1997) und *Aspergillus niger* (Conesa et al. 2001; Conesa et al. 2001) ist gar nicht oder nur mit sehr geringen Ausbeuten möglich. Es gibt zwar Versuche, gerichtet Mutationen in *C. fumago* einzuführen, wie z. B. in Rai et al. 2001; van de Velde et al. 2001; Rai et al. 2000; Yi et al. 1999; oder Zong 1997, aber eine Steigerung der Ausbeute an CPO in *C. fumago* wurde nicht versucht.

### Aufgabe

Aufgabe der Erfindung ist es, ein Expressionssystem sowie ein Verfahren zur Expression von homologen Proteinen und/oder Peptiden in einem Pilz der Klasse *Dothideomycetes,* insbesondere *C. fumago,* bereitzustellen, welches geeignet ist, natürlicherweise in diesem Organismus vorkommende , insbesondere nur in unzureichender Menge gebildete Proteine, insbesondere in erhöhter Menge, zur Expression zu bringen.

### Lösung

Diese Aufgabe wird durch die Erfindungen mit den Merkmalen der unabhängigen Ansprüche gelöst. Vorteilhafte Weiterbildungen der Erfindungen sind in den Unteransprüchen gekennzeichnet. Der Wortlaut sämtlicher Ansprüche wird hiermit durch Bezugnahme zum Inhalt dieser Beschreibung gemacht. Die Erfindung umfasst auch alle sinnvollen und insbesondere alle erwähnten Kombinationen von unabhängigen und/oder abhängigen Ansprüchen.

Die Erfindung betrifft ein Verfahren zur Expression von homologen Proteinen und/oder Peptiden in einem Pilz der Klasse *Dothideomycetes,* bevorzugt der Ordnung *Capnodiales,* besonders bevorzugt der Familie der *Capnodiaceae.*

Im Folgenden werden einzelne Verfahrensschritte näher beschrieben. Die Schritte müssen nicht notwendigerweise in der angegebenen Reihenfolge durchgeführt werden, und das zu schildernde Verfahren kann auch weitere, nicht genannte Schritte aufweisen.

Ein Protein im Sinne der vorliegenden Erfindung ist ein Protein oder ein Teil davon (Peptid). Die Funktion des Proteins kann struktureller, katalytischer (Enzym), regulatorischer oder sonstiger Art sein. Das Protein ist homologen Ursprungs in Bezug auf den zur Expression verwendeten Pilz. Es kann sich auch um mehrere Proteine handeln. Im Folgenden wird das Verfahren für ein einzelnes Protein erläutert. Die das Protein kodierende Sequenz kann Abweichungen von der in dem Organismus natürlicherweise vorkommenden Sequenz haben, beispielsweise durch Hinzufügen, Entfernen oder Verändern von Sequenzabschnitten. Durch gezielte Einführung von Sequenzänderungen kann Einfluss auf die Transkription, den Transport, die Sekretion, die posttranslationale Modifikation oder andere das Protein bzw. dessen Gensequenz betreffende Prozesse und Eigenschaften genommen werden. Homolog im Sinne der Erfindung bedeutet, dass die codierende Nukleotidsequenz für das Protein oder Peptid mindestens eine Sequenzidentität von mindestens 70 % zur entsprechenden Sequenz des natürlich im Organismus vorkommenden Proteins oder Peptid aufweist, bevorzugt ist eine Ähnlichkeit von über 80 %, besonders bevorzugt von 90 bis 100 %.

In einem ersten Schritt werden die Pilzzellen des Pilzes der Klasse *Dothideomycetes* mit einem für die mehrfache genomische Integration geeigneten Vektor transformiert. Dazu eignet sich jeder beliebige Klonierungsvektor, der DNA Fragmente aufnehmen und mit dem man eine Pilzzelle transformieren kann. Geeignete Vektoren sind dem Fachmann bekannt und verfügbar. Vor der Transformation kann es nötig sein, den Vektor mittels eines Restriktionsverdaus zu linearisieren.

Der Vektor enthält eine Expressionskassette, welche mindestens eine für das Protein und/oder Peptid kodierenden Gensequenz und ein als Selektionsmarker wirkendes Gen enthält. Ein solcher Selektionsmarker ist ein Gen, welches die Selektion spezifisch derjenigen Pilzzellen erlaubt, welche nach einem Transformationsschritt den Vektor, insbesondere die Expressionskassette, in ihr Genom integriert haben und den Selektionsmarker in hinreichender Stärke exprimieren. Als Selektionsmarker kann eine Vielzahl von Markergenen verwendet werden, welche zur Transformation von Pilzen geeignet sind. Geeignete Selektionsmarker sind beispielsweise Gene, die dem Pilz Resistenz gegenüber Antibiotika verleihen, z.B. gegen Hygromycin B, G418 und weitere Substanzen. Neben Antibiotikaresistenzgenen können auch Auxotrophie-Marker wie beispielsweise argB, trpC oder pyrG sowie weitere Methoden Verwendung finden. Die Liste genannter Selektionsmarker ist exemplarisch zu verstehen. Dem Fachmann sind weitere geeignete Selektionsmarker bekannt. In einer bevorzugten Ausführungsform der Erfindung findet das Hygromycin-Phosphotransferase Gen, welches Resistenz gegen das Antibiotikum Hygromycin B verleiht, als Selektionsmarker Verwendung.

Das zu exprimierende Protein der Expressionskassette muss in exprimierbarer Form kodiert vorliegen, das heißt, die DNA Sequenz muss neben den kodierenden Abschnitten auch geeignete Promotor-, Terminatorsequenzen und ggf. weitere zur Transkription erforderliche Sequenzen umfassen, die gewährleisten, dass eine Expression des Proteins in der bevorzugten Pilzart möglich ist. Bevorzugt sind diese Sequenzen homologen Ursprungs.

Der Vektor ist für mehrfache genomische Integration geeignet. Der Sequenzbereich des Vektors, welcher den Selektionsmarker und die Expressionskassette des zu exprimierenden Protein enthält, ist daher flankiert von DNA Sequenzen, welche sequenzidentisch oder hinreichend sequenzähnlich (Sequenzidentität von über 70%, bevorzugt über 80 %, über 90 %) mit genomischen Sequenzabschnitten des Zielorganismus sind, um die stabile genomische Integration der Expressionskassette über homologe Rekombination zu ermöglichen. Die Länge der zu einer genomischen Sequenz homologen Rekombinationssequenz beträgt mindestens 0,1kB (100 Basenpaare), bevorzugt mindestens 0,2 kB, besonders bevorzugt mindestens 0,5 kB, insbesondere zwischen 0,5 kB und 2 kB. Die Anzahl der in das Genom integrierten Expressionskassetten hängt davon ab, wie oft die homologen Abschnitte im Genom des Zielorganismus vorkommen. Dies kann abhängig von der Sequenz bis zu mehrere hundert Mal der Fall sein. Allerdings kann die Anzahl der integrierten Expressionskassetten auch von der Rekombinationsrate der entsprechenden Bereiche im Genom abhängen. Der Einbau der Expressionskassette kann beispielsweise durch PCR untersucht werden. Ebenso ist es möglich, durch quantitative PCR oder Southern-Blot die Anzahl der integrierten Expressionskassetten festzustellen.

Klone, die die Expressionskassette und den Selektionsmarker stabil in ihr Genom integriert haben, erhalten durch den Selektionsmarker einen Überlebensvorteil, z.B. mittels des Hygromycin-Phosphotransferase Gens Resistenz gegen das Antibiotikum Hygromycin B. Der Selektionsmarker ermöglicht dadurch die gezielte Selektion derjenigen Pilzzellen, deren Genom die Expressionskassette und den Selektionsmarker enthält. Im Falle von auxotrophen Selektionsmarkern können die Zellen auch über ein entsprechendes Minimalmedium selektiert werden.

Die selektierten Pilzzellen, welche die Expressionskassette in ihr Genom integriert haben, werden danach unter Expression des Proteins und/oder Peptids kultiviert. Dies kann beispielsweise durch die Initiierung eines auf der Expressionskassette enthaltenen Promotors geschehen.

In einem letzten Schritt wird das Protein und/oder Peptid gereinigt. Dazu kann es nötig sein, die Pilzzellen aufzuschließen und/oder das Protein und/oder Peptid mit einer Markersequenz, beispielsweise einem Affinitätsmarker, zu exprimieren. Es können auch andere dem Fachmann bekannte Methoden zu Aufreinigung von Proteinen oder Peptiden verwendet werden.

In einer Weiterbildung der Erfindung handelt es sich bei dem Pilz um *Caldariomyces fumago.*

In einer bevorzugten Weiterbildung der Erfindung wird das exprimierte Protein und/oder Peptid durch den Pilz sezerniert. Gegebenenfalls kann das Protein und/oder Peptid durch Anpassung der Sequenz der Expressionskassette durch eine entsprechende Signalsequenz ergänzt werden.

In einer Weiterbildung der Erfindung handelt es sich bei dem in der Expressionskassette codierten und zu exprimierenden Protein um ein Enzym mit Peroxidaseaktivität, bevorzugt um eine Chloroperoxidase.

In einer weiteren bevorzugten Ausführungsform der Erfindung handelt es sich bei dem in der Expressionskassette codierten und zu exprimierenden Protein um ein Protein, was mindestens 70 %, 80 %, 90 %, 95 % oder gar 100 % Sequenzidentität mit der *Caldariomyces fumago* eigenen Chloroperoxidase (CPO) aufweist. Dieses Enzym wird bei einer Vielzahl von chemischen Reaktionen eingesetzt und ist daher von besonderem Interesse. Außerdem wird dieses Enzym durch den Pilz sezerniert.

In einer weiteren bevorzugten Ausführungsform der Erfindung enthält die Expressionskassette als zu exprimierendes Protein eine Sequenz, welche mindestens 70 %, 80 %, 90 %, 95 % oder 100 % Sequenzidentität mit Seq-ID Nr. 1 aufweist.

In einer weiteren bevorzugten Ausführungsform der Erfindung enthält die Expressionskassette als zu exprimierendes Protein eine Sequenz, welche mindestens 70 %, 80 %, 90 %, 95 % oder 100 % Sequenzidentität mit Seq-ID Nr. 3 aufweist.

Mit dem erfindungsgemäßen Verfahren ist es möglich, beispielsweise bei homologen Proteinen, eine Erhöhung der Ausbeute an exprimiertem Protein und/oder Peptid um über 20 %, bevorzugt über 50 % im Vergleich zum Wildtyp (DSM 1256) zu erzielen. Übliche Werte für das erfindungsgemäße Verfahren liegen zwischen 20 % und 200 %, bevorzugt zwischen 20 % und 100 %. Im Falle eines sezernierten Proteins bestimmt sich die Ausbeute nach der maximalen Konzentration im Kulturmedium.

In einer bevorzugten Ausführungsform der Erfindung ist der Vektor geeignet, die Expressionskassette in ribosomale DNA (rDNA) zu integrieren.

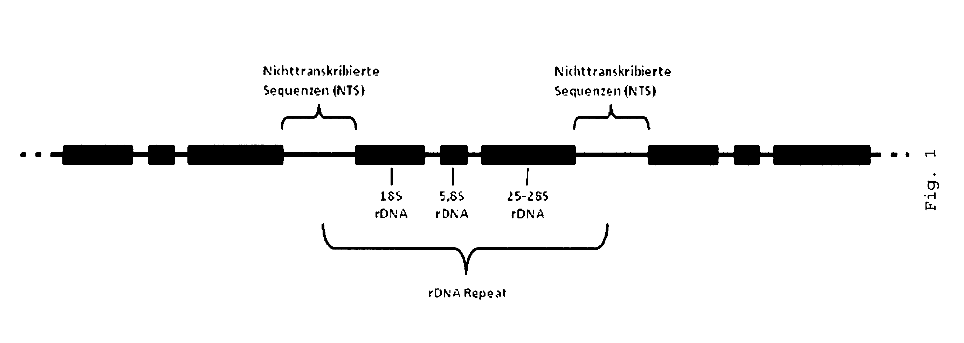

Ribosomale DNA (rDNA) bezeichnet genomische Sequenzbereiche, welche ribosomale RNA kodieren. Die transkriptionalen Einheiten des Ribosoms sind im Genom in der Form von Tandemrepeats angeordnet, d.h. die einzelnen Einheiten der RNA sind in immer gleicher Reihenfolge angeordnet. Von diesen Einheiten können in einem Genom mehrere Hundert vorliegen. Zwischen den Tandemrepeats liegen nicht transkribierte Bereiche (NTS: Nichttranskibierte Sequenzen), welche sich ebenfalls wiederholen. Figur 1 zeigt beispielsweise einen solchen rDNA-Repeat, welcher mehrere ribosomale Einheiten (18S, 5.8S, 25-28S) enthält. Zwischen diesen Sequenzen sind die NTS-Bereiche.

Um die Expressionskassette in die ribosomale DNA zu integrieren, müssen die flankierenden Sequenzen homolog zu Abschnitten der ribosomalen DNA sein.

Mit Vorteil ist der Vektor dafür geeignet, die Expressionskassette in die nicht transkribierten Bereiche der ribosomalen DNA zu integrieren. Die Verwendung nicht-kodierender Bereiche hat den Vorteil, dass dadurch verhindert wird, dass durch Insertion zusätzlicher DNA Sequenzen in ribosomale Gene die Gesamtkonzentration funktionsfähiger ribosomaler DNA in der Zelle abnimmt und die Zelle an Fitness verliert, wodurch auch die Proteingewinnung aus einem entsprechenden Pilzstamm beeinträchtigt sein könnte.

In einer weiteren bevorzugten Ausführungsform der Erfindung werden mindestens 2, bevorzugt mehr als 4, besonders bevorzugt zwischen 4 und 7 kurze Palindromsequenzen (z.B. TCGATCGA) in der Expressionskassette, bevorzugt im Promotorbereich des zu exprimierenden Proteins, eingefügt. Die Palindromsequenzen sind bevorzugt zwischen 5 und 9 Basen lang, bevorzugt zwischen 6 und 8.

In einer weiteren Ausführungsform der Erfindung enthält die Expressionskassette, bevorzugt im Promotorbereich des zu exprimierenden Proteins, eine Sequenz gemäß SEQ-ID Nr. 4, welche 6 palindromische Sequenzen enthält.

Die Erfindung betrifft außerdem einen Vektor zur genomischen Integration einer Gensequenz zu Expression eines Proteins und/oder Peptids in einen Pilz der Klasse *Dothideomycetes.* Ein solcher Vektor enthält mindestens eine Expressionskassette wie bereits vorstehend beschrieben und mindestens zwei Sequenzen, welche zur ribosomalen DNA des Zielorganismus homolog sind. Die verschiedenen Sequenzen können durch weitere Sequenzen voneinander getrennt sein und der Vektor kann noch weitere Sequenzen enthalten. Entscheidend ist, dass durch homologe Rekombination der zur ribosomalen DNA des Zielorganismus homologen Sequenzen, die Expressionskassette in das Genom des Zielorganismus integriert wird. Dazu muss die Expressionskassette von mindestens zwei dieser Sequenzen flankiert werden. Mit Vorteil handelt es sich um Sequenzen aus den nicht transkribierten Bereichen der rDNA des Zielorganismus.

Diese Bereiche haben dabei eine Länge von über 100 Basen, bevorzugt über 500 Basen. In einer bevorzugten Ausführungsform haben sie eine Länge von 100 bis 2000 Basen, bevorzugt zwischen 500 und 1500 Basen, besonders bevorzugt zwischen 600 und 1200 Basen. Dabei können längere homologe Bereiche, insbesondere Bereiche mit einer Sequenzähnlichkeit von über 90 %, die Spezifizität der Integration erhöhen.

In einer bevorzugten Weiterbildung ist der Zielorganismus *Caldariomyces fumago.*

In einer weiteren Weiterbildung der Erfindung ist die Sequenz der zur ribosomalen DNA des Zielorganismus homologen Sequenzen ausgewählt aus der SEQ-ID Nr. 5, besonders bevorzugt aus der nicht transkribierten rDNA SEQ-ID Nr. 6, beispielsweise wie Sequenzen der Seq-ID Nr. 9 und Seq-ID Nr. 10)

In einer Weiterbildung der Erfindung enthält die Expressionskassette eine Sequenz, welche mindestens 70 %, 80 %, 90 %, 95 % oder 100 % Sequenzidentität mit Seq-ID Nr. 1-Gen aufweist.

In einer Weiterbildung der Erfindung enthält die Expressionskassette eine Sequenz, welche mindestens 70 %, 80 %, 90 %, 95 % oder 100 % Sequenzidentität mit Seq-ID Nr. 2 aufweist.

In einer Weiterbildung der Erfindung umfasst die Expressionskassette eine Sequenz, welche mindestens 70 %, 80 %, 90 %, 95 % oder 100 % Sequenzidentität mit Seq-ID Nr. 7 aufweist.

In einer Weiterbildung der Erfindung umfasst der Vektor eine Sequenz, welche mindestens 70 %, 80 %, 90 %, 95 % oder 100 % Sequenzidentität mit Seq-ID Nr. 8 aufweist, welche eine Expressionskassette flankiert von homologen Bereichen aus NTS-Bereichen enthält.

Der Vektor kann auch eine veränderte Promotorsequenz, wie im Verfahren beschrieben aufweisen.

Ein solcher Vektor wird bevorzugt in dem erfindungsgemäßen Verfahren verwendet.

Die Erfindung betrifft außerdem einen genveränderten Stamm von *Caldariomyces fumago,* der eine für ein Protein oder Peptid kodierende Gensequenz stabil in die Sequenz seiner ribosomalen DNA integriert hat.

In einer vorteilhaften Weiterbildung der Erfindung ist die für ein Protein oder Peptid kodierende Gensequenz in die nicht transkribierten Bereiche der ribosomalen DNA des genveränderten Stammes integriert. Bevorzugt weist die ribosomale DNA eine Sequenzidentität von über 70 %, 80 %, 90 %, 95 % oder 100 % zu der Seq-ID Nr. 5, bevorzugt zu Seq-ID Nr. 6 auf. Bevorzugt besitzen diese nichttranskribierten Bereiche des genveränderten Stammes, in denen keine Sequenz eines Proteins oder Peptids integriert ist, eine Sequenzidentität von über 70 %, 80 %, 90 %, 95 % oder 100 % zu der Seq-ID Nr. 5, bevorzugt Seq-ID Nr. 6. Dies bedeutet, dass in einen oder mehreren Repeats der ribosomalen DNA die kodierende Gensequenz in die nichttranskribierten rDNA integriert ist, während die Bereiche der nichttranskribierten rDNA ohne integrierte Gensequenz eine Sequenzidentität von über 70 %, 80 %, 90 %, 95 % oder 100 % zu der Seq-ID Nr. 5, bevorzugt Seq-ID Nr. 6, aufweisen. Vor der Integration wiesen erstere Bereiche natürlich ebenfalls eine Sequenzidentität von über 70 %, 80 %, 90 %, 95 % oder 100 % zu der Seq-ID Nr. 5, bevorzugt Seq-ID Nr. 6, auf. Die integrierte Sequenz wird somit von Teilen der Sequenzen mit einer Sequenzidentität von über 70 %, 80 %, 90 %, 95 % oder 100 % zu der Seq-ID Nr. 5, bevorzugt Seq-ID Nr. 6, flankiert, ähnlich wie für den Vektor beschrieben.

In einer vorteilhaften Weiterbildung codiert die in das Genom integrierte Sequenz ein Protein oder Peptid mit Peroxidaseaktivität.

In einer vorteilhaften Weiterbildung weist die in das Genom integrierte Gensequenz mindestens 70 %, 80 %, 90 %, 95 % oder 100 % Sequenzidentität zu zu Seq-ID Nr. 1 auf.

In einer vorteilhaften Weiterbildung weist die in das Genom integrierte Gensequenz mindestens 70 %, 80 %, 90 %, 95 % oder 100 % Sequenzidentität zu zu Seq-ID Nr. 2 auf.

Mit Vorteil ist eine wie vorstehend beschriebene Expressionskassette in die nicht transkribierten Bereiche der ribosomalen DNA integriert.

Die Erfindung betrifft außerdem einen gentechnisch veränderten Stamm von *Caldariomyces fumago,* der mit einem erfindungsgemäßen Vektor transformiert wurde.

Ein erfindungsgemäß gentechnisch veränderter Stamm zeigt bei der genomischen Integration eines homologen Proteins oder Peptids eine um mindestens 20 %, 30 % oder 50 % höhere Expression dieses Proteins oder Peptids im Vergleich zum Wildtyp. Mit Vorteil handelt es sich bei dem homologen Protein um eine Chloroperoxidase, bevorzugt um die *Caldariomyces fumago* eigene Chloroperoxidase.

Die Erfindung betrifft außerdem eine Expressionskassette, wie bei dem Verfahren und für den Vektor beschrieben.

Die Erfindung betrifft auch die Verwendung eines erfindungsgemäßen gentechnisch veränderten Stamms von *Caldariomyces fumago* zur Expression von Proteinen und Peptiden, bevorzugt von Proteinen, welche eine Peroxidaseaktivität aufweisen.

Weitere Einzelheiten und Merkmale ergeben sich aus der nachfolgenden Beschreibung von bevorzugten Ausführungsbeispielen in Verbindung mit den Unteransprüchen. Hierbei können die jeweiligen Merkmale für sich alleine oder zu mehreren in Kombination miteinander verwirklicht sein. Die Möglichkeiten, die Aufgabe zu lösen, sind nicht auf die Ausführungsbeispiele beschränkt. So umfassen beispielsweise Bereichsangaben stets alle - nicht genannten - Zwischenwerte und alle denkbaren Teilintervalle.

Die Ausführungsbeispiele sind in den Figuren schematisch dargestellt. Gleiche Bezugsziffern in den einzelnen Figuren bezeichnen dabei gleiche oder funktionsgleiche bzw. hinsichtlich ihrer Funktionen einander entsprechende Elemente. Im Einzelnen zeigt:
- Fig. 1: Schema vom Aufbau rDNA-Repeats;
- Fig. 2: Vektor pGEM-Cf2,7;
- Fig. 3: Vektor pBS-NTS3/5;
- Fig. 4: pBS-NTS3/5-Hph;
- Fig. 5: Vektor pBS-NTS3/5-Hph-CPO; und
- Fig. 6: Vergleich der CPO-Expression für Wildtyp und gentech- nisch veränderten Stamm *C. fumago.*

### Identifizierung der nichttranskribierten rDNA-Sequenzen von C. fumago

Mittels PCR-Reaktionen wurde unter Verwendung von genomischer *C. fumago*-DNA und jeweils zwei Primerkombinationen zunächst zwei Fragmente der *C. fumago*-rDNA amplifiziert, in einen *E. coli*-Vektor kloniert und deren Sequenz durch DNA-Sequenzierung ermittelt.

Primerkombination 1:
NS1 5'-GTAGTCATATGCTTGTCTC (Seq-ID Nr. 11) und
NS2 5'-GGCTGCTGGCACCAGACTTGC (Seq-ID Nr. 12)

Primerkombination 2:
LR10R 5'-GACCCTGTTGAGCTTGA (Seq-ID Nr. 13) und
LR12 5'-GACTTAGAGGCGTTCAG (Seq-ID Nr. 14)

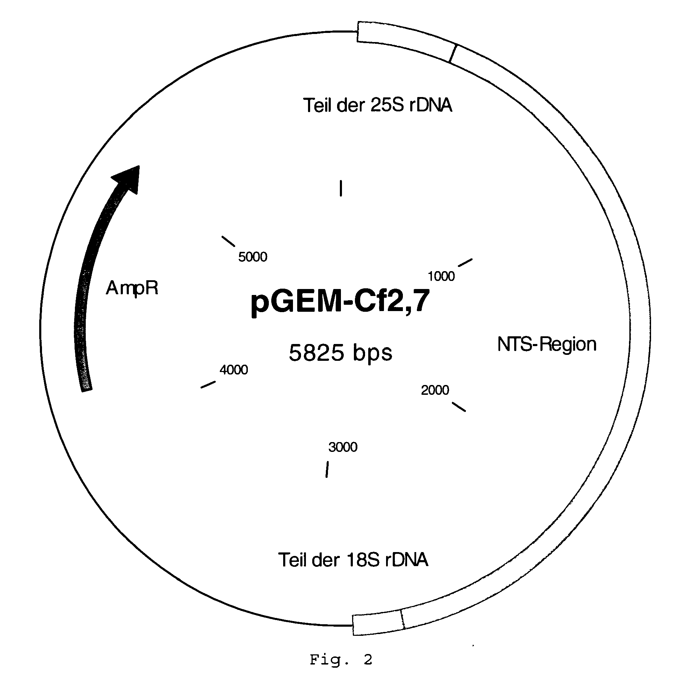

Ausgehend von den so erhaltenen Sequenzinformationen der 18S rDNA-Region und der 25S rDNA-Region von *C. fumago* wurde mit einem weiteren Primerpaar:
LR-MB1: 5'-AGTGCCGCGAAGCTACCATCTG (Seq-ID Nr. 15) und
SR-MB1R: 5'-TTACCACGGTTATCCATGTAGTAAGG (Seq-ID Nr. 16)
eine weitere PCR mit genomischer *C. fumago*-DNA als Template durchgeführt. Das so erhaltene DNA-Fragment, das in einen *E. coli*-Vektor (pGEM-T von Firma Promega) kloniert und dessen Sequenz durch DNA-Sequenzierung ermittelt wurde, enthielt neben Teilen der 18S rDNA-Region und der 25S rDNA-Region von *C. fumago* auch die gesamte Sequenz des nichttranskribierten Bereichs der *C. fumago*-rDNA. Die Sequenz dieser Bereiche ist in SEQ-ID Nr. 5, bzw. Seq-ID Nr. 6, angegeben. Dieser Vektor (pGEM-Cf2,7, Fig. 2) diente dann als Matrize für PCR-Reaktionen bei der Konstruktion des *C. fumago* Integrationsvektors.

### Konstruktion des Integrationsvektors

Um einen Vektor zu erzeugen, mit dessen Hilfe bestimmte DNA-Sequenzen in den nichttranskribierten Bereich der *C. fumago-*rDNA eingebracht werden können, wurden zunächst in zwei nacheinander folgenden Schritten zwei Fragmente des nichttranskribierten Bereichs der *C. fumago*-rDNA in den Vektor pBluescript KS(+) der Firma Stratagene eingebracht.

In einem ersten Schritt wurde das NTS3 benannte Fragment des nichttranskribierten Bereichs der *C. fumago*-rDNA mittels einer PCR-Reaktion mit den Primern
Cf-NTS3-Not5 5'-TGCAGCGGCCGCGGTATTGCCACTTCTCGACAC (Seq-ID Nr. 17) und
Cf-NTS3-Sac3 5'-TGCAGAGCTCGACCACCTCCTTGGGAGTTTC (Seq-ID Nr. 18)
und dem Vektor pGEM-Cf2,7 als Template amplifiziert (Seq-ID Nr. 9). Nach einer Inkubation des erhaltenen PCR-Fragments und auch des Ausgangsvektors pBluescript KS(+) mit den beiden Restriktionsenzymen SacI und NotI wurden die beiden linearen Fragmente mit einer Ligase inkubiert und der Reaktionsansatz anschließend in *E. coli* DH5α transformiert. Nach einer Selektion auf entsprechendem Plattenmedium wurde Plasmid-DNA aus mehreren Klonen isoliert und mittels Restriktionsverdaus analysiert. Auf diese Weise konnte das Plasmid pBS-NTS3 erhalten werden, das bereits ein Fragment des nichttranskribierten Bereichs der *C. fumago-*rDNA enthält.

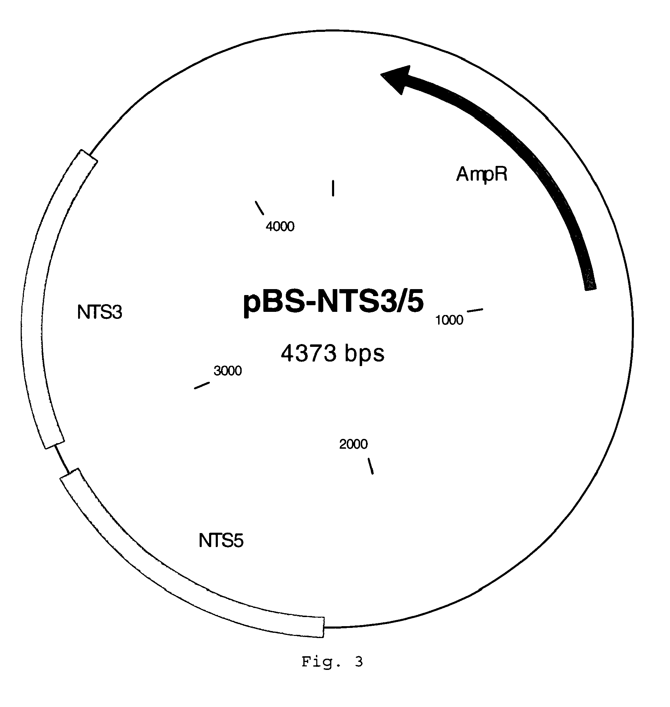

Im zweiten Schritt wurde das NTS5 benannte Fragment des nichttranskribierten Bereichs der *C. fumago*-rDNA mittels einer PCR-Reaktion mit den Primern
Cf-NTS5-Kpn5 5'-TGCAGGTACCTCAGTTCAAAAGCCCATGCCTG (Seq-ID Nr. 19)und
Cf-NTS5-Sal3 5'-TGCAGTCGACGATACACGGCAAGTGAGCAAC (Seq-ID Nr. 20)
und dem Vektor pGEM-Cf2,7 als Template amplifiziert (Seq-ID Nr. 10). Nach einer Inkubation des erhaltenen PCR-Fragments und des Vektors pBS-NTS3 mit den beiden Restriktionsenzymen KpnI und SalI wurden die beiden linearen Fragmente mit einer Ligase inkubiert und der Reaktionsansatz anschließend in *E. coli* DH5α transformiert. Nach einer Selektion auf entsprechendem Plattenmedium wurde Plasmid-DNA aus mehreren Klonen isoliert und mittels Restriktionsverdaus analysiert. Das so erhaltene Plasmid pBS-NTS3/5 enthielt zwei Fragmente des nichttranskribierten Bereichs der *C. fumago*-rDNA (Fig. 3).

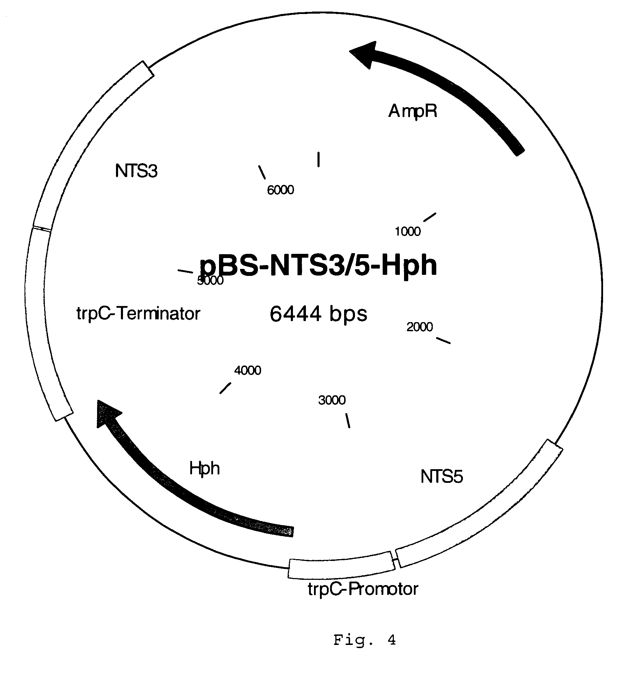

Nach dem Einbringen der für die Integration in die rDNA-Region notwendigen DNA-Fragmente wurde in einem weiteren Schritt ein DNA-Fragment in den Vektor eingefügt, das Pilzzellen Resistenz gegenüber dem Antibiotikum Hygromycin B verleiht und deshalb nach einer Transformation in *C. fumago*-Zellen eine Selektion auf solche Zellen ermöglicht, die den Integrationsvektor stabil in das Genom integriert haben. Dazu wurde das Hygromycin-Phosphotransferase-Gen von *E. coli* und Bereiche des Promotors und des Terminators des TrpC-Gens von *Aspergillus nidulans* mittels einer PCR-Reaktion mit den Primern
Hph-XbaI-Hind5 5'-ACTGAAGCTTAGATCTCTAGAGACAGAAGATGATATTG (Seq-ID Nr. 21) und
Hph-Not3 5'-AGCTGCGGCCGCACAAGTGTACCTGTGCATTCTG (Seq-ID Nr. 22)
und dem Vektor pCSN43 (Staben et al. 1989) als Template amplifiziert. Nach einer Inkubation des erhaltenen PCR-Fragments und des Vektors pBS-NTS3/5 mit den beiden Restriktionsenzymen HindIII und NotI wurden die beiden linearen Fragmente mit einer Ligase inkubiert und der Reaktionsansatz anschließend in *E. coli* DH5α transformiert. Nach einer Selektion auf entsprechendem Plattenmedium wurde Plasmid-DNA aus mehreren Klonen isoliert und mittels Restriktionsverdaus analysiert. Das so erhaltene Plasmid pBS-NTS3/5-Hph-Xba enthielt die Hygromycin-Phosphotransferase-Expressionskassette zwischen den beiden Fragmenten des nichttranskribierten Bereichs der *C. fumago*-rDNA (Fig. 4).

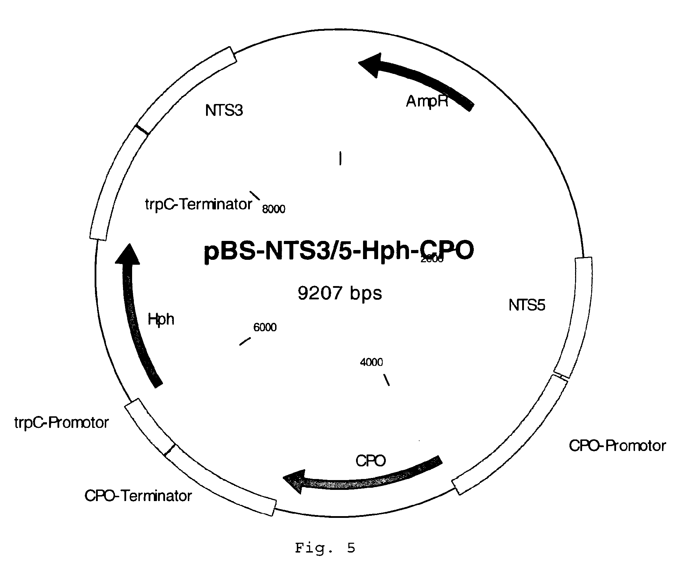

Im letzten Schritt der Vektorkonstruktion wurde das CPO-Gen von *C. fumago* mit dem nativen Promotor und Terminator in den Integrationsvektor eingebracht. Dazu wurde der entsprechende Bereich des *C. fumago*-Genoms mittels einer PCR-Reaktion mit den Primern
CPO-Hind5 5'-ACTGAAGCTTCTGGTTTGCATGCACTTGCATC (Seq-ID Nr. 23)und
CPO-Xba3 5'-ACTGTCTAGATGCCTTATACTAACACTCAGATCAG (Seq-ID Nr. 24)
und mit genomischer *C. fumago*-DNA als Template amplifiziert. Die Primer wurden dabei auf der Grundlage der bekannten Sequenz des CPO-Gens sowie des Promotor- und Terminator-Bereichs (Nuell et al. 1988) ausgewählt. Nach einer Inkubation des erhaltenen PCR-Fragments und des Vektors pBS-NTS3/5-Hph-Xba mit den beiden Restriktionsenzymen HindIII und XbaI wurden die beiden linearen Fragmente mit einer Ligase inkubiert und der Reaktionsansatz anschließend in *E. coli* DH5α transformiert. Nach einer Selektion auf entsprechendem Plattenmedium wurde Plasmid-DNA aus mehreren Klonen isoliert und mittels Restriktionsverdaus analysiert. Das so erhaltene Plasmid pBS-NTS3/5-Hph-CPO enthielt zwischen den beiden Fragmenten des nichttranskribierten Bereichs der *C. fumago*-rDNA sowohl die Hygromycin-Phosphotransferase-Expressionskassette als auch die CPO-Expressionskassette (Fig. 5).

### Transformation des Integrationsvektors in C. fumago-Sphäroblasten

Um die CPO-Expressionskassette in den rDNA-Bereich des *C. fumago*-Genoms zu integrieren, wurde das Plasmid pBS-NTS3/5-Hph-CPO zunächst durch eine Inkubation mit dem Restriktionsenzym KpnI linearisiert und anschließend mittels Elektroporation in *C. fumago*-Sphäroblasten transformiert.

Für die Linearisierung des zu transformierenden Plasmids wurden 100µl einer Lösung des Vektors pBS-NTS3/5-Hph-CPO (∼0,5µg/µl) mit 6µl KpnI-Enzymlösung (10 Units/µl; New England Biolabs R0142S) und dem mitgelieferten Puffer der Firma New England Biolabs für 6h bei 37°C inkubiert. Von dem so linearisierten Plasmid wurde nach Anwendung des "PCR Purification Kit" (Roche) 50µl der gereinigten DNA in dem im "PCR Purification Kit" enthaltenen Puffer EB erhalten.

Für die Herstellung von *C. fumago*-Sphäroblasten wurde zunächst der Stamm *C. fumago* (DSM 1256) in einem Schüttelkolben mit 50ml Kartoffelmedium (30g/L Glukose; 200g/L gekochte und anschließend pürierte Kartoffeln) unter ständigem Schütteln für 5 Tage bei 24°C inkubiert. Das Pilzmycel wurden nach dieser Anzucht durch Zentrifugation geerntet (10min; 4000g; 24°C) und zweimal mit Sphäroblastierungspuffer (1,2M MgSO₄; 10mM Kaliumphosphat-Puffer pH 5,8) gewaschen. Anschließend wurde das Pilzmycel in 50ml Sphäroblastierungspuffer resuspendiert und nach Zugabe von 0,6g einer zellwandabbauenden Enzymmischung von Trichoderma harzianum (Sigma Aldrich L1412) unter leichtem Schütteln 2h45min bei 24°C inkubiert. Nach Abzentrifugieren der Zellpellets (1min; 150g; 24°C) wurde der Überstand zweimal durch sterile Glaswolle gefiltert, um einzellige Sphäroblasten zu erhalten, während größere Pilzstrukturen größtenteils im Glaswolle-Filter verbleiben. Die Sphäroblasten wurden dann durch Zentrifugation (5min; 1000g; 24°C) geerntet und nach Entfernen des Überstands in 15ml Transformationspuffer (10mM Bis/Tris-Puffer pH6,5; 1,2M Sorbitol; 10mM CaCl₂) resuspendiert. Nach erneuter Zentrifugation (5min; 1000g; 24°C) wurden die Sphäroblasten noch mal in 15ml Transformationspuffer resuspendiert unter wieder unter den gleichen Bedingungen zentrifugiert. Nach Entfernen des Überstands wurden die Sphäroblasten in 200µl Transformationspuffer resuspendiert. 80µl der Sphäroblastenlösung wurden mit 5µl des linearisierten und gereinigten Plasmids pBS-NTS3/5-Hph-CPO gemischt und in eine Elektroporationsküvette mit einem Elektrodenabstand von 2mm gefüllt. Nach der Elektroporation (1000V; 25µF; 300Ω) mit dem Gerät "Gene Pulser Xcell" (BioRad) wurde 1ml Regenerationsmedium (1M Saccharose; 5g/L Pepton; 1g/L Hefeextrakt; 24°C) zu den transformierten Zellen gegeben und diese Lösung 30h bei 24°C in einer sterilen Petrischale inkubiert. Dann wurde die Zellsuspension auf Selektionsmedium (40g/L Fructose; 20g/L Malzextrakt; 2g/L NaNO₃; 2g/L KCl; 2g/L KH₂PO₄; 1g/L MgSO₄ * 7H₂O; 20mg/L FeSO₄ * 7H₂O; 500mg/L Hygromycin B) aufgebracht und bei 24°C inkubiert. Nach vier Tagen wurden mehrere Kolonien isoliert, auf Selektionsmedium aufgebracht und weitere fünf Tage bei 24°C inkubiert. Aus dem so erhaltenen Mycel wurde genomische DNA isoliert und für PCR-Reaktionen zum Nachweis der Integration des Plasmids in der rDNA-Region des *C. fumago*-Genoms eingesetzt. Dazu wurden die Primerkombination
Cf-25S-1 5'-AGCCCGTTGTCGGCTCGATTTG (Seq-ID Nr. 25) und
MCS-1 5'-AAGCTTATCGATACCGTCGACG (Seq-ID Nr. 26)
und die Primerkombination
TrpC-Term-1 5'-TCGTTTACCCAGAATGCACAGG (Seq-ID Nr. 27) und
NTS3-1 5'-AACCACCAAGGCGTCTACCTG (Seq-ID Nr. 28)
verwendet. Der Klon CPO4, mit dessen DNA mit beiden Primerkombinationen ein PCR-Produkt der jeweils erwarteten Länge erhalten werden konnte, wurde für eine Kultivierung im Bioreaktor verwendet.

In wie vielen Kopien das CPO-Gen in dem Genom des Überexpressionsstamms CPO4 vorliegt, wurde nicht bestimmt, evtl. ist also auch nur eine zusätzliche Kopie eingebracht worden.

### Kultivierung im Bioreaktor

Jeweils 100ml Kartoffelmedium (30g/L Glukose; 200g/L gekochte und anschließend pürierte Kartoffeln) in Schüttelkolben wurden mit 1cm² Mycel des Stamms CPO4 von einer Selektionsmedium-Agarplatte bzw. des Wildtypstamms *C. fumago* (DSM 1256) von einer PDA-Agarplatte (Difco 254920) inokuliert und unter Schütteln bei 24°C inkubiert. Nach 7 Tagen wurden die Zellpellets in dieser Kultur durch eine 20 Sekunden lange Behandlung (Stufe 3) mit einem Homogenisator (Ultra Turrax T25 basic; IKA Labortechnik) zerkleinert. Zwei Bioreaktoren eines 1,5L Multifermentationssystems mit Überkopfrührung (DASGIP) wurden mit jeweils 50g Fructose und 800ml H₂O befüllt und anschließend autoklaviert. Nach Abkühlen der Lösung wurden jeweils 170ml einer sterilfiltrierten Salz-Lösung (2g KH₂PO₄; 2g NaNO₃; 2g KCl; 1g MgSO₄ x 7 H₂O; 20mg FeSO₄ x 7 H₂O) und 30ml der homogenisierten Vorkulturen zur Inokulation zugegeben. Die Rührergeschwindigkeit während der Kultivierung betrug 300rpm, die Belüftungsrate mit gefilterter Luft 0,1vvm und die Temperatur 24°C. Alle zwei Tage wurden 10ml einer hochkonzentrierten Fructoselösung (100g Fructose gemischt mit 50ml H₂O) zugegeben.

Vom fünften bis zum 14. Tag der Kultivierung wurde jeweils 1ml Kultur entnommen und nach Zentrifugation (20sec; 14.100g) der zellfreie Überstand abgenommen und für die Bestimmung der CPO-Aktivität eingesetzt. Dazu wurde der MCD-Enzymtest (Hager et al. 1966) eingesetzt, wobei die Abnahme der Absorption bei 278nm bestimmt und mit Hilfe des molaren Extinktionskoeffizienten von MCD (Monochlorodimedon) bei 278 nm (12.200 M⁻¹cm⁻¹) die Enzymaktivität berechnet wurde. Mit Hilfe der spezifischen Aktivität gereinigter CPO von 1660U/mg (Morris und Hager 1966) wurde die Enzymkonzentration im Kulturüberstand berechnet.

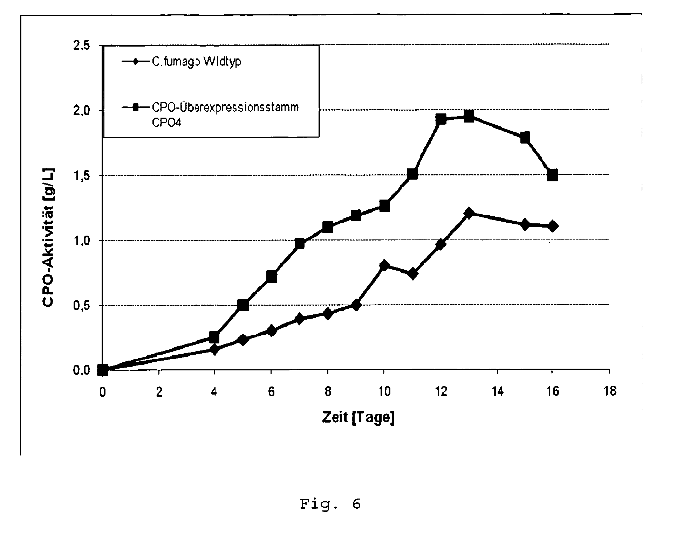

Figur 6 zeigt einen Vergleich der CPO-Aktivität in g/L des selektierten Klons im Vergleich zu dem Wildtyp. Bei der Kultivierung wurde eine Aktivität von bis zu 1.95 g/L CPO gemessen, während der Wildtyp nur bis zu 1.21 g/L produzierte. Dies ist der höchste bisher gemessene Wert für die Produktion von CPO mit *C. fumago.*

Es sind zahlreiche Abwandlungen und Weiterbildungen der beschriebenen Ausführungsbeispiele verwirklichbar.

Liste der zitierten Literatur:
Conesa, A., F. van De Velde, F. van Rantwijk, R. A. Sheldon, C. A. van Den Hondel und P. J. Punt (2001). "Expression of the Caldariomyces fumago chloroperoxidase in Aspergillus niger and characterization of the recombinant enzyme." J Biol Chem 276(21): 17635-40.
Conesa, A., G. Weelink, C. A. van den Hondel und P. J. Punt (2001). "C-terminal propeptide of the Caldariomyces fumago chloroperoxidase: an intramolecular chaperone?" FEBS Lett 503(2-3): 117-20.
Hager, L. P., D. R. Morris, F. S. Brown und H. Eberwein (1966). "Chloroperoxidase. II. Utilization of halogen anions." J Biol Chem 241(8): 1769-77.
Morris, D. R. und L. P. Hager (1966). "Chloroperoxidase I. Isolation and properties of the crystalline glycoprotein." The Journal of Biological Chemistry 241(8): 1763-1768.
Nuell, M. J., G. H. Fang, M. J. Axley, P. Kenigsberg und L. P. Hager (1988). "Isolation and nucleotide sequence of the chloroperoxidase gene from Caldariomyces fumago." J Bacteriol 170(2): 1007-11.
Rai, G., S. Sakai, A. Flórez, L. Mogollon und L. Hager (2001). "Directed Evolution of Chloroperoxidase for Improved Epoxidation and Chlorination Catalysis." Adv. Synth. Catal. 343(6-7): 638-645.
Rai, G. P., Q. Zong und L. P. Hager (2000). "Isolation of Directed Evolution Mutants of Chloroperoxidase Resistant to Suicide Inactivation by Primary Olefins." Israel Journal of Chemistry 40: 63-70.
Staben, C., B. Jensen, M. Singer, J. Pollock, M. Schechtman, J. Kinsey und E. Selker (1989). "Use of a bacterial Hygromycin B resistance gene as a dominant selectable marker in Neurospora crassa transformation." Fungal Genetics Newsl. 36: 79-81.
van deVelde, F., M. Bakker, F. Rantwijk van, G. P. Rai und L. P. Hager (2001). "Engineering chloroperoxidase for activity and stability." Journal of Molecular Catalysis B: Enzymatic 11: 765-769.
Yi, X., M. Mroczko, K. M. Manoj, X. Wang und L. P. Hager (1999). "Replacement of the proximal heme thiolate ligand in chloroperoxidase with a histidine residue." Proc Natl Acad Sci U S A 96(22): 12412-7.
Zong, Q. (1997). "Expression of recombinant chloroperoxidase." PhD thesis, Graduate College of the University of Illinois at Urbana-Champaign. (p. 52-110)

## Patentansprüche

1. Verfahren zur Expression von homologen Proteinen und/oder Peptiden davon in einem Pilz der Klasse *Dothideomycetes* mit den Schritten:
a) Transformation von Pilzzellen des Pilzes mit einem für die mehrfache genomische Integration geeigneten Vektor, wobei
b) der Vektor eine Expressionskassette enthält, welche mindestens eine Gensequenz für
b1) mindestens ein homologes Protein und/oder Peptid und
b2) mindestens ein als Selektionsmarker wirkendes Gen enthält;
c) Selektion von Pilzzellen, welche die Expressionskassette stabil in ihr Genom integriert haben;
d) Kultivierung der Pilzzellen unter Expression des Proteins und/oder Peptids der Expressionskassette;
e) Reinigung des Proteins und/oder Peptids.

2. Verfahren nach Anspruch 1,
**dadurch gekennzeichnet, dass**
es sich bei dem Pilz um *Caldariomyces* fumago handelt.

3. Verfahren nach Anspruch 1 oder 2,
**dadurch gekennzeichnet, dass**
es sich bei dem für die mehrfache genomische Integration geeigneten Vektor um einen Vektor handelt, der für die mehrfache genomische Integration in ribosomale DNA geeignet ist, wobei der Vektor für die mehrfache genomische Integration in die nicht transkribierten Bereiche der ribosomalen DNA geeignet ist.

4. Verfahren nach einem der vorhergehenden Ansprüche,
**dadurch gekennzeichnet, dass**
die Gensequenz für ein Protein und/oder Peptid mit Peroxidaseaktivität, insbesondere eine Chloroperoxidase, kodiert.

5. Verfahren nach einem der vorhergehenden Ansprüche, **dadurch gekennzeichnet, dass**
die Gensequenz für ein Protein und/oder Peptid mindestens 70 % Sequenzidentität zu SEQ-ID Nr. 1 aufweist.

6. Vektor zur genomischen Integration einer Gensequenz in einen Pilz der Klasse *Dothideomycetes,* **dadurch gekennzeichnet, dass**
der Vektor eine Expressionskassette enthält, welche mindestens eine Sequenz für
a) mindestens ein homologes Protein und/oder Peptid,
b) mindestens ein als Selektionsmarker wirkendes Gen enthält und der Vektor
c) mindestens zwei zur ribosomalen DNA des Pilzes homologe Sequenzen aufweist, wobei diese Sequenzen die Expressionskassette flankieren.

7. Vektor nach Anspruch 6, **dadurch gekennzeichnet, dass** die mindestens zwei zur ribosomalen DNA des Pilzes homologen Sequenzen jeweils aus der Sequenz SEQ-ID Nr. 5 ausgewählt sind und jeweils mindestens eine Länge von 100 Basen aufweisen.

8. Vektor nach einem der Ansprüche 6 oder 7, **dadurch gekennzeichnet, dass**
die Sequenz für mindestens ein Protein und/oder Peptid mindestens 70 % Sequenzidentität mit SEQ-ID Nr. 1-Gen aufweist.

9. Vektor nach einem der Ansprüche 6 bis 8,
**dadurch gekennzeichnet, dass**
die Sequenz der Expressionskassette mindestens 70 % Sequenzidentität zur SEQ-ID Nr. 2 aufweist.

10. Gentechnisch veränderter Stamm von *Caldariomyces fumago*, **dadurch gekennzeichnet, dass**
er eine ein Protein und/oder Peptid kodierende Gensequenz stabil in die Sequenz seiner ribosomalen DNA integriert hat.

11. Gentechnisch veränderter Stamm von *Caldariomyces fumago* nach Anspruch 10, **dadurch gekennzeichnet, dass**
er eine für ein Protein und/oder Peptid kodierende Gensequenz stabil in die nicht transkribierten Bereiche seiner ribosomalen DNA integriert hat.

12. Gentechnisch veränderter Stamm von *Caldariomyces fumago* nach einem der Ansprüche 10 oder 11, **dadurch gekennzeichnet, dass**
die Gensequenz ein homologes Protein und/oder Peptid mit Peroxidaseaktivität kodiert.

13. Gentechnisch veränderter Stamm von *Caldariomyces fumago* nach Anspruch 12, **dadurch gekennzeichnet, dass**
er eine Sequenz mit mindestens 70 % Sequenzidentität zu SEQ-ID Nr. 1 in seine ribosomale DNA integriert hat.

14. Gentechnisch veränderter Stamm von *Caldariomyces fumago*, transformiert mit einem Vektor nach einem der Ansprüche 6 bis 9.

15. Expressionskassette mit einer eine Peroxidaseaktivität kodierenden Sequenz, wie im Stamm nach Anspruch 12 enthalten.

16. Expressionskassette nach Anspruch 15, **dadurch gekennzeichnet, dass**
die Expressionskassette eine Gensequenz gemäß SEQ ID Nr. 1 aufweist.

17. Verwendung eines Stammes nach einem der Ansprüche 10 bis 14 zur Expression von homologen Proteinen und/oder Peptiden.


SEQUENCE LISTING

<110> DECHEMA Gesellschaft für chemische Technik und
      Biotechnologie e.V.

<120> Verfahren zur Expression von homologen Proteinen und/oder
      Peptiden in Pilzen der Klasse Dothideomycetes

<130> DECPT09071

<160> 28

<170> PatentIn version 3.3

<210> 1
<211> 1122
<212> DNA
<213> Caldariomyces fumago


<220>
<221> gene
<222> (1)..(1122)
<223> CPO

<220>
<221> CDS
<222> (1)..(1122)
<223> CPO

<220>
<221> sig_peptide
<222> (1)..(63)
<223> N-terminales Signalpeptid

<220>
<221> misc_feature
<222> (964)..(1122)
<223> C-terminales Propeptid

<400> 1
atg ttc tcc aag gtc ctt ccc ttc gtg gga gcg gtt gcc gcc ctc cct      48
Met Phe Ser Lys Val Leu Pro Phe Val Gly Ala Val Ala Ala Leu Pro
1               5                   10                  15

cac tcc gtc cgt cag gag cct ggc tcc ggc att ggc tac cca tac gac      96
His Ser Val Arg Gln Glu Pro Gly Ser Gly Ile Gly Tyr Pro Tyr Asp
            20                  25                  30

aac aac acc ctg cca tat gtc gcc cca ggt cct acc gac tct cgt gct      144
Asn Asn Thr Leu Pro Tyr Val Ala Pro Gly Pro Thr Asp Ser Arg Ala
        35                  40                  45

cct tgc cca gct ctg aac gct ctt gcc aac cac ggt tac att cct cac      192
Pro Cys Pro Ala Leu Asn Ala Leu Ala Asn His Gly Tyr Ile Pro His
    50                  55                  60

gat ggc cgt gcc atc agc agg gag acc ctc cag aac gct ttc ctc aac      240
Asp Gly Arg Ala Ile Ser Arg Glu Thr Leu Gln Asn Ala Phe Leu Asn
65                  70                  75                  80

cac atg ggt att gcc aac tcc gtc att gag ctt gct ctg acc aac gcc      288
His Met Gly Ile Ala Asn Ser Val Ile Glu Leu Ala Leu Thr Asn Ala
                85                  90                  95

ttc gtc gtc tgc gag tac gtt act ggc tcc gac tgt ggt gac agc ctt      336
Phe Val Val Cys Glu Tyr Val Thr Gly Ser Asp Cys Gly Asp Ser Leu
            100                 105                 110

gtc aac ctg act ctg ctc gcc gag ccc cac gct ttc gag cac gac cac 384
Val Asn Leu Thr Leu Leu Ala Glu Pro His Ala Phe Glu His Asp His
        115                 120                 125

tcc ttc tcc cgc aag gat tac aag cag ggt gtc gcc aac tcc aac gac 432
Ser Phe Ser Arg Lys Asp Tyr Lys Gln Gly Val Ala Asn Ser Asn Asp
    130                 135                 140

ttc atc gac aac agg aac ttc gat gcc gag acc ttc cag acc tct ctg 480
Phe Ile Asp Asn Arg Asn Phe Asp Ala Glu Thr Phe Gln Thr Ser Leu
145                 150                 155                 160

gat gtc gtt gca ggc aag acc cac ttc gac tat gcc gac atg aac gag 528
Asp Val Val Ala Gly Lys Thr His Phe Asp Tyr Ala Asp Met Asn Glu
                165                 170                 175

atc cgc ctt cag cgc gag tcc ctc tcc aac gag ctt gac ttc ccc ggt 576
Ile Arg Leu Gln Arg Glu Ser Leu Ser Asn Glu Leu Asp Phe Pro Gly
            180                 185                 190

tgg ttc acc gag tcc aag cca atc cag aac gtc gag tct ggc ttc atc 624
Trp Phe Thr Glu Ser Lys Pro Ile Gln Asn Val Glu Ser Gly Phe Ile
        195                 200                 205

ttc gcc ctt gtc tct gac ttc aac ctg ccc gac aac gat gag aac cct 672
Phe Ala Leu Val Ser Asp Phe Asn Leu Pro Asp Asn Asp Glu Asn Pro
    210                 215                 220

ctg gtt cgc att gac tgg tgg aag tac tgg ttc acc aac gag tcc ttc 720
Leu Val Arg Ile Asp Trp Trp Lys Tyr Trp Phe Thr Asn Glu Ser Phe
225                 230                 235                 240

cca tac cac ctc ggc tgg cac ccc ccg tct cca gcc agg gag atc gag 768
Pro Tyr His Leu Gly Trp His Pro Pro Ser Pro Ala Arg Glu Ile Glu
                245                 250                 255

ttc gtc acc tcc gcc tcc tcc gct gtc ctg gct gcc tct gtc acc tct 816
Phe Val Thr Ser Ala Ser Ser Ala Val Leu Ala Ala Ser Val Thr Ser
            260                 265                 270

act cca tct tcc ctt cca tcc ggt gcc atc ggc cca ggt gcc gag gct 864
Thr Pro Ser Ser Leu Pro Ser Gly Ala Ile Gly Pro Gly Ala Glu Ala
        275                 280                 285

gtc cct ctc tcc ttc gcc tcc acc atg acc cca ttc ctc ctc gcc acc 912
Val Pro Leu Ser Phe Ala Ser Thr Met Thr Pro Phe Leu Leu Ala Thr
    290                 295                 300

aat gct cct tac tac gcc cag gac cca act ctc ggc ccc aac gac aag 960
Asn Ala Pro Tyr Tyr Ala Gln Asp Pro Thr Leu Gly Pro Asn Asp Lys
305                 310                 315                 320

cgt gag gct gcc cca gct gcc acc acc tcc atg gcc gtc ttc aag aac 1008
Arg Glu Ala Ala Pro Ala Ala Thr Thr Ser Met Ala Val Phe Lys Asn
                325                 330                 335

cca tac ctc gag gcc att ggc acc cag gac atc aag aac cag cag gct 1056
Pro Tyr Leu Glu Ala Ile Gly Thr Gln Asp Ile Lys Asn Gln Gln Ala
            340                 345                 350

tac gtc agc tcc aag gct gct gcc atg gcc tct gcc atg gcc gcc aac 1104
Tyr Val Ser Ser Lys Ala Ala Ala Met Ala Ser Ala Met Ala Ala Asn
        355                 360                 365

aag gcc cgc aac ctt taa 1122
Lys Ala Arg Asn Leu
    370

<210>  2
<211>  373
<212>  PRT
<213>  Caldariomyces fumago

<400>  2

Met Phe Ser Lys Val Leu Pro Phe Val Gly Ala Val Ala Ala Leu Pro
1               5                   10                  15

His Ser Val Arg Gln Glu Pro Gly Ser Gly Ile Gly Tyr Pro Tyr Asp
            20                  25                  30

Asn Asn Thr Leu Pro Tyr Val Ala Pro Gly Pro Thr Asp Ser Arg Ala
        35                  40                  45

Pro Cys Pro Ala Leu Asn Ala Leu Ala Asn His Gly Tyr Ile Pro His
    50                  55                  60

Asp Gly Arg Ala Ile Ser Arg Glu Thr Leu Gln Asn Ala Phe Leu Asn
65                  70                  75                  80

His Met Gly Ile Ala Asn Ser Val Ile Glu Leu Ala Leu Thr Asn Ala
                85                  90                  95

Phe Val Val Cys Glu Tyr Val Thr Gly Ser Asp Cys Gly Asp Ser Leu
            100                 105                 110

Val Asn Leu Thr Leu Leu Ala Glu Pro His Ala Phe Glu His Asp His
        115                 120                 125

Ser Phe Ser Arg Lys Asp Tyr Lys Gln Gly Val Ala Asn Ser Asn Asp
    130                 135                 140

Phe Ile Asp Asn Arg Asn Phe Asp Ala Glu Thr Phe Gln Thr Ser Leu
145                 150                 155                 160

Asp Val Val Ala Gly Lys Thr His Phe Asp Tyr Ala Asp Met Asn Glu
                165                 170                 175

Ile Arg Leu Gln Arg Glu Ser Leu Ser Asn Glu Leu Asp Phe Pro Gly
            180                 185                 190

Trp Phe Thr Glu Ser Lys Pro Ile Gln Asn Val Glu Ser Gly Phe Ile
        195                 200                 205

Phe Ala Leu Val Ser Asp Phe Asn Leu Pro Asp Asn Asp Glu Asn Pro
    210                 215                 220

Leu Val Arg Ile Asp Trp Trp Lys Tyr Trp Phe Thr Asn Glu Ser Phe
225                 230                 235                 240

Pro Tyr His Leu Gly Trp His Pro Pro Ser Pro Ala Arg Glu Ile Glu
                245                 250                 255

Phe Val Thr Ser Ala Ser Ser Ala Val Leu Ala Ala Ser Val Thr Ser
            260                 265                 270

Thr Pro Ser Ser Leu Pro Ser Gly Ala Ile Gly Pro Gly Ala Glu Ala
        275                 280                 285

Val Pro Leu Ser Phe Ala Ser Thr Met Thr Pro Phe Leu Leu Ala Thr
    290                 295                 300

Asn Ala Pro Tyr Tyr Ala Gln Asp Pro Thr Leu Gly Pro Asn Asp Lys
305                 310                 315                 320

Arg Glu Ala Ala Pro Ala Ala Thr Thr Ser Met Ala Val Phe Lys Asn
                325                 330                 335

Pro Tyr Leu Glu Ala Ile Gly Thr Gln Asp Ile Lys Asn Gln Gln Ala
            340                 345                 350

Tyr Val Ser Ser Lys Ala Ala Ala Met Ala Ser Ala Met Ala Ala Asn
        355                 360                 365

Lys Ala Arg Asn Leu
    370

<210>  3
<211>  2770
<212>  DNA
<213>  Caldariomyces fumago

<220>
<221>  promotor
<222>  (1)..(927)

<220>
<221>  gene
<222>  (928)..(2049)
<223>  CPO

<220>
<221>  sig_peptide
<222>  (928)..(990)
<223>  N-terminales Signalpeptid

<220>
<221>  misc_feature
<222>  (1891)..(2049)
<223>  C-terminales Propeptid

<220>
<221>  terminator
<222>  (2050)..(2770)

<400>  3
tctggtttgc atgcacttgc atcgcctgcc tgtttgcatc cacaaccaga cgcttcaaga      60

agtgatcagc tccaaatggc aacctgtgtt cgcattagcc tcactcgggc gctgtgcagc 120
gcagtgcagc gcactgttgc attacctgtt ggcaaaggac cgccctgacc gttgtggcta 180
aggtttcgtc ctactctgtg cctgcaacct aaaactcgcc gcagagctca agcctgcgga 240
ctaccctctc tccaccactg atgcttcacg atccacatcg tgtttagtga tggccctgtt 300
cctgacctga cgtagcgact attctgggca aatccttcga cagtaagcca tctctgtgcc 360
ttgtgcccct ccccgtggcc ctcgcgcgca gcataccctc ccttgtggtg cgactccaac 420
atgaagctag cacgtgtaag caaggcaatc ctcacctctg ttcactaatt cgtgtcgctc 480
tgcaactcca tactgatcag acaatgccag agctggtggt caacccaacg aacggtctta 540
cgatcctgct gacctaccgc ctcactagtc ctaccgggag agttcgactg acttgcagca 600
gactgcccac ggaacctgtc tgctcgctgt agagatggtg atgagtgatg gtcgatatgc 660
cgacatcctt ctctcgttcg accctagtgc tctaccgata cggacgccct tcgactcagc 720
caaaggaggc atttggactt ggacaaagca gaacatatcg cctctcgatc gatataagta 780
tcagcatctc ctcctgccat aattcaactt tctcctcatc atccaacaaa ccatccaatc 840
ctctaaagca tctggctcac aacaagcctc tgctcattca ttcgctctaa gtctttgaat 900
cttcgattct tcgatacttc tctcgctatg ttctccaagg tccttccctt cgtgggagcg 960
gttgccgccc tccctcactc cgtccgtcag gagcctggct ccggcattgg ctacccatac 1020
gacaacaaca ccctgccata tgtcgcccca ggtcctaccg actctcgtgc tccttgccca 1080
gctctgaacg ctcttgccaa ccacggttac attcctcacg atggccgtgc catcagcagg 1140
gagaccctcc agaacgcttt cctcaaccac atgggtattg ccaactccgt cattgagctt 1200
gctctgacca acgccttcgt cgtctgcgag tacgttactg gctccgactg tggtgacagc 1260
cttgtcaacc tgactctgct cgccgagccc cacgctttcg agcacgacca ctccttctcc 1320
cgcaaggatt acaagcaggg tgtcgccaac tccaacgact tcatcgacaa caggaacttc 1380
gatgccgaga ccttccagac ctctctggat gtcgttgcag gcaagaccca cttcgactat 1440
gccgacatga acgagatccg ccttcagcgc gagtccctct ccaacgagct tgacttcccc 1500
ggttggttca ccgagtccaa gccaatccag aacgtcgagt ctggcttcat cttcgccctt 1560
gtctctgact tcaacctgcc cgacaacgat gagaaccctc tggttcgcat tgactggtgg 1620
aagtactggt tcaccaacga gtccttccca taccacctcg gctggcaccc cccgtctcca 1680
gccagggaga tcgagttcgt cacctccgcc tcctccgctg tcctggctgc ctctgtcacc 1740
tctactccat cttcccttcc atccggtgcc atcggcccag gtgccgaggc tgtccctctc 1800
tccttcgcct ccaccatgac cccattcctc ctcgccacca atgctcctta ctacgcccag 1860
gacccaactc tcggccccaa cgacaagcgt gaggctgccc cagctgccac cacctccatg 1920
gccgtcttca agaacccata cctcgaggcc attggcaccc aggacatcaa gaaccagcag 1980
gcttacgtca gctccaaggc tgctgccatg gcctctgcca tggccgccaa caaggcccgc 2040
aacctttaag cgcatctacg acatcgagat ggtccagcat tcactgttcc gaatgtgaat 2100

gtgctactta acgacaacac gaggaaatgc ctttcctcac tacagcatca gcatgcagag 2160

gcgcatttgg tttttcccgc atagatcggc tggcttggtc acgagttttc cgattgcacg 2220

attgatttta tagaatagaa cgatagacta tagacataga aaaaactgta tgcatttcat 2280

gccgcgtttt accccaaaac ctatctcctc tcttttcgcc gtctattttc tcaattccct 2340

ctttcgtttc ttcccctttg gttttacaga caatcaatca atcgatcgat cgaaaggctc 2400

attggccttc ttacatttac tgttatccct tccgcattgg tacagaacag tgtggaggaa 2460

cgcggtatga gatcataacc atcattacaa catcatggca gcgcagatcg ctgcgagctt 2520

cttacccagg aaaggatcgg ggtatcatta agctaccgcc ctggcctagc cagtcgcccg 2580

aagatctagc ttgcagcggc tgccctattt tctcacccat gcgggccatg actgttcgcg 2640

aagttagtcc cctatccaag ttgaaaagat gggaaagtgg agatttctta cgtcgtgttg 2700

actacttttg ccacttccct tatcagacgt atatactcca acatctgatc tgagtgttag 2760

tataaggcat 2770

<210> 4
<211> 209
<212> DNA
<213> künstliche Sequenz

<400> 4
gatcagacaa tgccagagct ggtggtcaac ccaacgaacg gtcttacgat cctgctgacc 60

taccgcctct cgatcgaagt ctcgatcgag cattcgatcg atcgatcgat cgatgaatcg 120

atcgaacggt cgatcgatat aagtatcagc atctcctcct gccataattc aactttctcc 180

tcatcatcca acaaaccatc caatcctct 209

<210> 5
<211> 2823
<212> DNA
<213> Caldariomyces fumago

<220>
<221> misc_feature
<222> (1)..(307)
<223> Teil der 25S rDNA

<220>
<221> misc_feature
<222> (308)..(2669)
<223> Nicht transkribierter Bereich der rDNA

<220>
<221> misc_feature
<222> (2670)..(2823)
<223> Teil der 18S rDNA

<400> 5
agtgccgcga agctaccatc tgctggatta tggctgaacg cctctaagtc agaatccatg 60

ccggaacggg gcgatcctct ccagcacgtc gtacgagtgt acgaataggc ctttggcccc 120

ggaccccagc aggggcgcgg tccgcatacc tagaagtggt gtgcggtggc gcccggattg 180

caatttgaca gcgcgctgga atgaatcctt tgtagacgac ttagccgtcc gaccgggtcg 240
tgtaagcagt cgagtagcct tgttgttacg agctgctgag cgtaagcccg ttgtcggctc 300
gatttgtcct ccccccatcg ttgtgtcccc ggcagagagc cgggtacccg gagggggtct 360
cagttcaaaa gcccatgcct gctcccagtc tgggggcagt ttgggtgtgg agggatcggg 420
gcagggccat ccccggcgcg agatacactt gggctccacg acccttgggt gacagagctg 480
cgcagcaggt agacaggtag accagagacc ggagaccggg cctgcctgcc catgcggtcc 540
gccacagggg gatttggagg aaggaacgaa gcccagggcg caggtacagg tgtgtgcccc 600
tcgaagccgc gagggtggcc actggggcag agtaggctgg agagcgtggg aggtgagact 660
ggtgccgcat ggagctgggc cgcgacagcc accacctccc acatggagga agcgagctgc 720
tggcagcggt gcgcgttggg gtccgggcag ggccaccccc acgtgccaca gccccattgc 780
ggcgttggga ggcggcgttg ggagatgagg cagggtcgcc cgggacggcg ttgggctcgg 840
ggcagggtca ccccgtgagg ggctgcagct acaggcggct tgctctggtc ccggacattc 900
tgggacctct tacctctgac ctccgagctc ttgcctctgc cctctggcgt tggcattctt 960
tggggcccgt ccagcgctcc ggccgcggcg ttggcagggg cctggcggcg ttggcatcag 1020
ggctcagccc tccagccgtg gcgttggggg gcagcggacc ggttgctcac ttgccgtgta 1080
tcgtcatggg gcagaaggga gaggtgggtg gacaaccgaa ccaatacacg gcaggacaga 1140
gaccgcccct ggagagtaca agggtggacc atgaggaagg gtacctggcg gccctggcga 1200
gaagtgtggt gtttgaggag ggaagggagg gggacaaacc aagagagaga cgaggatcga 1260
ggctcgtctc cctgaggaac cgccctcacg gcatagtctc gtaccgggta ttgccacttc 1320
tcgacacaca cacacacacc ggccacccta caggccgtct acacctcctc tctggagcga 1380
ggacaacatg agacaatcac agatgggggg gaggaatagg gaagcaacga ggctgccagc 1440
ggcggaaggc agaaggcggt gcgtgccctt tcccaatctg gcggcggcag cgaggtgggc 1500
gggcaatgta ctgggaggca tgggagcggc cacccagatg gctccagagg gcctgggagg 1560
ggtgtgtgag gcgtgtgtga ccaagtcagt gagtgcgcgc agtttcccag cacccggcga 1620
tgcgcgcgcg cgcgtctctc gcaggcccgg caccaaaacg gtgtatgcgt gggacgcact 1680
tttccggggg cggctcgccg cacacgagga tcaaagtcca tgacgttcgg aggatatata 1740
agaagaaagc gtctcacagt ttcagaaaaa gcgtaaaaac cttttctctg gagcgatgtc 1800
ccctgatgga aggtcgtaat gggctagcat ctgggggccc taggcgctct ctatggcggg 1860
cctcctggcc tcgactttga tgggttctgg ggtgttggta gtcgcggggc aacccgtttg 1920
actcccccca aaggtccccc ctgcaggatg ctgcatgcgc aggcacacgc ttcgagtcac 1980
tcggcgaggt gttgcgcccc gaaactccca aggaggtggt cggccgttca ggtagacgcc 2040
ttggtggttg aggggcgcga gcgcatgtag gatccagcgg gaggtggact gccccggcga 2100
agccgtggcc tgccgccgca atagcgaggc cccaagcgag cctgcgtcct aactagcact 2160
gcctagcgct gctaggacgc ctgcgagcct gagggccaac gtccccatgg aagccagctt 2220

gattggcggt ccggccggcc tcaccgccgg ctgtcgaccc cgacagcagc aacctggggg 2280

cgtcagaagc ccggctccct ccctggaggt gagccggcaa gggtggagtc tgtggatcgc 2340

tggccgttgg ctatcgctgt ttccagacgc actcgggggt acgcctatgt gatggtatcc 2400

ggctcgtccg tgaccattga tgggtagcct cctcacgagg ccgccatgcc acagatgggt 2460

cccgccttaa ctggatgggg tcctcctgcg gcggaagcgg ccttggggaa gtccagtggg 2520

ggcaaatgga tggccgagcg ccaaacccgt acgggcatcg ccccgggtaa ggcaccggcc 2580

ggggactcaa tgtctcccct ccaagttatc taactccggg cccctccatc gcggtggggt 2640

tcagggccat cgtgcccgcc gaagatagtt acctggttga ttctgccagt agtcatatgc 2700

ttgtctcaaa gattaagcca tgcatgtcta agtataagca actatacggt gaaactgcga 2760

atggctcatt aaatcagtta tcgtttattt gatagtacct tactacatgg ataaccgtgg 2820

taa 2823

<210> 6
<211> 2362
<212> DNA
<213> Caldariomyces fumago

<400> 6
cctcccccca tcgttgtgtc cccggcagag agccgggtac ccggaggggg tctcagttca 60

aaagcccatg cctgctccca gtctgggggc agtttgggtg tggagggatc ggggcagggc 120

catccccggc gcgagataca cttgggctcc acgacccttg ggtgacagag ctgcgcagca 180

ggtagacagg tagaccagag accggagacc gggcctgcct gcccatgcgg tccgccacag 240

ggggatttgg aggaaggaac gaagcccagg gcgcaggtac aggtgtgtgc ccctcgaagc 300

cgcgagggtg gccactgggg cagagtaggc tggagagcgt gggaggtgag actggtgccg 360

catggagctg ggccgcgaca gccaccacct cccacatgga ggaagcgagc tgctggcagc 420

ggtgcgcgtt ggggtccggg cagggccacc cccacgtgcc acagccccat tgcggcgttg 480

ggaggcggcg ttgggagatg aggcagggtc gcccgggacg gcgttgggct cggggcaggg 540

tcaccccgtg aggggctgca gctacaggcg gcttgctctg gtcccggaca ttctgggacc 600

tcttacctct gacctccgag ctcttgcctc tgccctctgg cgttggcatt ctttggggcc 660

cgtccagcgc tccggccgcg gcgttggcag gggcctggcg gcgttggcat cagggctcag 720

ccctccagcc gtggcgttgg ggggcagcgg accggttgct cacttgccgt gtatcgtcat 780

ggggcagaag ggagaggtgg gtggacaacc gaaccaatac acggcaggac agagaccgcc 840

cctggagagt acaagggtgg accatgagga agggtacctg gcggccctgg cgagaagtgt 900

ggtgtttgag gagggaaggg agggggacaa accaagagag agacgaggat cgaggctcgt 960

ctccctgagg aaccgccctc acggcatagt ctcgtaccgg gtattgccac ttctcgacac 1020

acacacacac accggccacc ctacaggccg tctacacctc ctctctggag cgaggacaac 1080

atgagacaat cacagatggg ggggaggaat agggaagcaa cgaggctgcc agcggcggaa 1140

ggcagaaggc ggtgcgtgcc ctttcccaat ctggcggcgg cagcgaggtg ggcgggcaat 1200

gtactgggag gcatgggagc ggccacccag atggctccag agggcctggg aggggtgtgt 1260

gaggcgtgtg tgaccaagtc agtgagtgcg cgcagtttcc cagcacccgg cgatgcgcgc 1320

gcgcgcgtct ctcgcaggcc cggcaccaaa acggtgtatg cgtgggacgc acttttccgg 1380

gggcggctcg ccgcacacga ggatcaaagt ccatgacgtt cggaggatat ataagaagaa 1440

agcgtctcac agtttcagaa aaagcgtaaa aaccttttct ctggagcgat gtcccctgat 1500

ggaaggtcgt aatgggctag catctggggg ccctaggcgc tctctatggc gggcctcctg 1560

gcctcgactt tgatgggttc tggggtgttg gtagtcgcgg ggcaacccgt ttgactcccc 1620

ccaaaggtcc cccctgcagg atgctgcatg cgcaggcaca cgcttcgagt cactcggcga 1680

ggtgttgcgc cccgaaactc ccaaggaggt ggtcggccgt tcaggtagac gccttggtgg 1740

ttgaggggcg cgagcgcatg taggatccag cgggaggtgg actgccccgg cgaagccgtg 1800

gcctgccgcc gcaatagcga ggccccaagc gagcctgcgt cctaactagc actgcctagc 1860

gctgctagga cgcctgcgag cctgagggcc aacgtcccca tggaagccag cttgattggc 1920

ggtccggccg gcctcaccgc cggctgtcga ccccgacagc agcaacctgg gggcgtcaga 1980

agcccggctc cctccctgga ggtgagccgg caagggtgga gtctgtggat cgctggccgt 2040

tggctatcgc tgtttccaga cgcactcggg ggtacgccta tgtgatggta tccggctcgt 2100

ccgtgaccat tgatgggtag cctcctcacg aggccgccat gccacagatg ggtcccgcct 2160

taactggatg gggtcctcct gcggcggaag cggccttggg gaagtccagt gggggcaaat 2220

ggatggccga gcgccaaacc cgtacgggca tcgccccggg taaggcaccg gccggggact 2280

caatgtctcc cctccaagtt atctaactcc gggcccctcc atcgcggtgg ggttcagggc 2340

catcgtgccc gccgaagata gt 2362

<210> 7
<211> 4879
<212> DNA
<213> künstliche Sequenz


<220>
<221> promotor
<222> (1)..(927)
<223> CPO-Promotor

<220>
<221> gene
<222> (928)..(2049)
<223> CPO

<220>
<221> sig_peptide
<222> (928)..(990)
<223> N-terminales Signalpeptid

<220>
<221> misc_feature
<222> (1891)..(2049)
<223> C-terminales Propeptid

<220>
<221>  terminator
<222>  (2050)..(2770)
<223>  CPO-Terminator

<220>
<221>  promotor
<222>  (2775)..(3142)
<223>  trpC-Terminator

<220>
<221>  gene
<222>  (3143)..(4168)
<223>  Hygromycin-Phosphotransferasegen (Selektionsmarker)

<220>
<221>  terminator
<222>  (4169)..(4879)
<223>  trpC-Terminator

<400>  7
tctggtttgc atgcacttgc atcgcctgcc tgtttgcatc cacaaccaga cgcttcaaga      60

agtgatcagc tccaaatggc aacctgtgtt cgcattagcc tcactcgggc gctgtgcagc     120

gcagtgcagc gcactgttgc attacctgtt ggcaaaggac cgccctgacc gttgtggcta     180

aggtttcgtc ctactctgtg cctgcaacct aaaactcgcc gcagagctca agcctgcgga     240

ctaccctctc tccaccactg atgcttcacg atccacatcg tgtttagtga tggccctgtt     300

cctgacctga cgtagcgact attctgggca aatccttcga cagtaagcca tctctgtgcc     360

ttgtgcccct ccccgtggcc ctcgcgcgca gcataccctc ccttgtggtg cgactccaac     420

atgaagctag cacgtgtaag caaggcaatc ctcacctctg ttcactaatt cgtgtcgctc     480

tgcaactcca tactgatcag acaatgccag agctggtggt caacccaacg aacggtctta     540

cgatcctgct gacctaccgc ctcactagtc ctaccgggag agttcgactg acttgcagca     600

gactgcccac ggaacctgtc tgctcgctgt agagatggtg atgagtgatg gtcgatatgc     660

cgacatcctt ctctcgttcg accctagtgc tctaccgata cggacgccct tcgactcagc     720

caaaggaggc atttggactt ggacaaagca gaacatatcg cctctcgatc gatataagta     780

tcagcatctc ctcctgccat aattcaactt tctcctcatc atccaacaaa ccatccaatc     840

ctctaaagca tctggctcac aacaagcctc tgctcattca ttcgctctaa gtctttgaat     900

cttcgattct tcgatacttc tctcgctatg ttctccaagg tccttccctt cgtgggagcg     960

gttgccgccc tccctcactc cgtccgtcag gagcctggct ccggcattgg ctacccatac    1020

gacaacaaca ccctgccata tgtcgcccca ggtcctaccg actctcgtgc tccttgccca    1080

gctctgaacg ctcttgccaa ccacggttac attcctcacg atggccgtgc catcagcagg    1140

gagaccctcc agaacgcttt cctcaaccac atgggtattg ccaactccgt cattgagctt    1200

gctctgacca acgccttcgt cgtctgcgag tacgttactg gctccgactg tggtgacagc    1260

cttgtcaacc tgactctgct cgccgagccc cacgctttcg agcacgacca ctccttctcc    1320

cgcaaggatt acaagcaggg tgtcgccaac tccaacgact tcatcgacaa caggaacttc    1380

gatgccgaga ccttccagac ctctctggat gtcgttgcag gcaagaccca cttcgactat 1440
gccgacatga acgagatccg ccttcagcgc gagtccctct ccaacgagct tgacttcccc 1500
ggttggttca ccgagtccaa gccaatccag aacgtcgagt ctggcttcat cttcgccctt 1560
gtctctgact tcaacctgcc cgacaacgat gagaaccctc tggttcgcat tgactggtgg 1620
aagtactggt tcaccaacga gtccttccca taccacctcg gctggcaccc cccgtctcca 1680
gccagggaga tcgagttcgt cacctccgcc tcctccgctg tcctggctgc ctctgtcacc 1740
tctactccat cttcccttcc atccggtgcc atcggcccag gtgccgaggc tgtccctctc 1800
tccttcgcct ccaccatgac cccattcctc ctcgccacca atgctcctta ctacgcccag 1860
gacccaactc tcggccccaa cgacaagcgt gaggctgccc cagctgccac cacctccatg 1920
gccgtcttca agaacccata cctcgaggcc attggcaccc aggacatcaa gaaccagcag 1980
gcttacgtca gctccaaggc tgctgccatg gcctctgcca tggccgccaa caaggcccgc 2040
aacctttaag cgcatctacg acatcgagat ggtccagcat tcactgttcc gaatgtgaat 2100
gtgctactta acgacaacac gaggaaatgc ctttcctcac tacagcatca gcatgcagag 2160
gcgcatttgg tttttcccgc atagatcggc tggcttggtc acgagttttc cgattgcacg 2220
attgatttta tagaatagaa cgatagacta tagacataga aaaaactgta tgcatttcat 2280
gccgcgtttt accccaaaac ctatctcctc tcttttcgcc gtctattttc tcaattccct 2340
ctttcgtttc ttcccctttg gttttacaga caatcaatca atcgatcgat cgaaaggctc 2400
attggccttc ttacatttac tgttatccct tccgcattgg tacagaacag tgtggaggaa 2460
cgcggtatga gatcataacc atcattacaa catcatggca gcgcagatcg ctgcgagctt 2520
cttacccagg aaaggatcgg ggtatcatta agctaccgcc ctggcctagc cagtcgcccg 2580
aagatctagc ttgcagcggc tgccctattt tctcacccat gcgggccatg actgttcgcg 2640
aagttagtcc cctatccaag ttgaaaagat gggaaagtgg agatttctta cgtcgtgttg 2700
actacttttg ccacttccct tatcagacgt atatactcca acatctgatc tgagtgttag 2760
tataaggcat ctagagacag aagatgatat tgaaggagca ctttttgggc ttggctggag 2820
ctagtggagg tcaacaatga atgcctattt tggtttagtc gtccaggcgg tgagcacaaa 2880
atttgtgtcg tttgacaaga tggttcattt aggcaactgg tcagatcagc cccacttgta 2940
gcagtagcgg cggcgctcga agtgtgactc ttattagcag acaggaacga ggacattatt 3000
atcatctgct gcttggtgca cgataacttg gtgcgtttgt caagcaaggt aagtgaacga 3060
cccggtcata ccttcttaag ttcgcccttc ctccctttat ttcagattca atctgactta 3120
cctattctac ccaagcatcg atatgaaaaa gcctgaactc accgcgacgt ctgtcgagaa 3180
gtttctgatc gaaaagttcg acagcgtctc cgacctgatg cagctctcgg agggcgaaga 3240
atctcgtgct ttcagcttcg atgtaggagg gcgtggatat gtcctgcggg taaatagctg 3300
cgccgatggt ttctacaaag atcgttatgt ttatcggcac tttgcatcgg ccgcgctccc 3360
gattccggaa gtgcttgaca ttggggaatt cagcgagagc ctgacctatt gcatctcccg 3420

ccgtgcacag ggtgtcacgt tgcaagacct gcctgaaacc gaactgcccg ctgttctgca 3480

gccggtcgcg gaggccatgg atgcgatcgc tgcggccgat cttagccaga cgagcgggtt 3540

cggcccattc ggaccgcaag gaatcggtca atacactaca tggcgtgatt tcatatgcgc 3600

gattgctgat ccccatgtgt atcactggca aactgtgatg gacgacaccg tcagtgcgtc 3660

cgtcgcgcag gctctcgatg agctgatgct ttgggccgag gactgccccg aagtccggca 3720

cctcgtgcac gcggatttcg gctccaacaa tgtcctgacg gacaatggcc gcataacagc 3780

ggtcattgac tggagcgagg cgatgttcgg ggattcccaa tacgaggtcg ccaacatctt 3840

cttctggagg ccgtggttgg cttgtatgga gcagcagacg cgctacttcg agcggaggca 3900

tccggagctt gcaggatcgc cgcggctccg ggcgtatatg ctccgcattg gtcttgacca 3960

actctatcag agcttggttg acggcaattt cgatgatgca gcttgggcgc agggtcgatg 4020

cgacgcaatc gtccgatccg gagccgggac tgtcgggcgt acacaaatcg cccgcagaag 4080

cgcggccgtc tggaccgatg gctgtgtaga agtactcgcc gatagtggaa accgacgccc 4140

cagcactcgt ccgagggcaa aggaatagag tagatgccga ccgggatcca cttaacgtta 4200

ctgaaatcat caaacagctt gacgaatctg gatataagat cgttggtgtc gatgtcagct 4260

ccggagttga gacaaatggt gttcaggatc tcgataagat acgttcattt gtccaagcag 4320

caaagagtgc cttctagtga tttaatagct ccatgtcaac aagaataaaa cgcgttttcg 4380

ggtttacctc ttccagatac agctcatctg caatgcatta atgcattgac tgcaacctag 4440

taacgccttc aggctccggc gaagagaaga atagcttagc agagctattt tcattttcgg 4500

gagacgagat caagcagatc aacggtcgtc aagagaccta cgagactgag gaatccgctc 4560

ttggctccac gcgactatat atttgtctct aattgtactt tgacatgctc ctcttcttta 4620

ctctgatagc ttgactatga aaattccgtc accagccctg ggttcgcaaa gataattgca 4680

tgtttcttcc ttgaactctc aagcctacag gacacacatt catcgtaggt ataaacctcg 4740

aaatcattcc tactaagatg gtatacaata gtaaccatgg ttgcctagtg aatgctccgt 4800

aacacccaat acgccggccg aaactttttt acaactctcc tatgagtcgt ttacccagaa 4860

tgcacaggta cacttgtgc 4879

<210> 8
<211> 6342
<212> DNA
<213> künstliche Sequenz


<220>
<221> misc_feature
<222> (1)..(723)
<223> NTS5

<220>
<221> promotor
<222> (744)..(1670)
<223> CPO-Promotor

<220>

<221>  gene
<222>  (1671)..(2792)
<223>  CPO-Gen

<220>
<221>  sig_peptide
<222>  (1671)..(1733)
<223>  N-terminales Signalpeptid

<220>
<221>  misc_feature
<222>  (2634)..(2792)
<223>  C-terminales Propeptid

<220>
<221>  terminator
<222>  (2793)..(3513)
<223>  CPO-Terminator

<220>
<221>  promotor
<222>  (3518)..(3885)
<223>  trpC-Promotor

<220>
<221>  gene
<222>  (3886)..(4911)
<223>  Hygromycin-Phosphotransferasegen

<220>
<221>  terminator
<222>  (4912)..(5622)
<223>  trpC-Terminator

<220>
<221>  misc_feature
<222>  (5631)..(6342)
<223>  NTS3

<400>  8
tcagttcaaa agcccatgcc tgctcccagt ctgggggcag tttgggtgtg gagggatcgg      60

ggcagggcca tccccggcgc gagatacact tgggctccac gacccttggg tgacagagct     120

gcgcagcagg tagacaggta gaccagagac cggagaccgg gcctgcctgc ccatgcggtc     180

cgccacaggg ggatttggag gaaggaacga agcccagggc gcaggtacag gtgtgtgccc     240

ctcgaagccg cgagggtggc cactggggca gagtaggctg gagagcgtgg gaggtgagac     300

tggtgccgca tggagctggg ccgcgacagc caccacctcc cacatggagg aagcgagctg     360

ctggcagcgg tgcgcgttgg ggtccgggca gggccacccc cacgtgccac agccccattg     420

cggcgttggg aggcggcgtt gggagatgag gcagggtcgc ccgggacggc gttgggctcg     480

gggcagggtc accccgtgag gggctgcagc tacaggcggc ttgctctggt cccggacatt     540

ctgggacctc ttacctctga cctccgagct cttgcctctg ccctctggcg ttggcattct     600

ttggggcccg tccagcgctc cggccgcggc gttggcaggg gcctggcggc gttggcatca     660

gggctcagcc ctccagccgt ggcgttgggg ggcagcggac cggttgctca cttgccgtgt     720

atcgtcgacg gtatcgataa gcttctggtt tgcatgcact tgcatcgcct gcctgtttgc     780

atccacaacc agacgcttca agaagtgatc agctccaaat ggcaacctgt gttcgcatta     840

gcctcactcg ggcgctgtgc agcgcagtgc agcgcactgt tgcattacct gttggcaaag 900
gaccgccctg accgttgtgg ctaaggtttc gtcctactct gtgcctgcaa cctaaaactc 960
gccgcagagc tcaagcctgc ggactaccct ctctccacca ctgatgcttc acgatccaca 1020
tcgtgtttag tgatggccct gttcctgacc tgacgtagcg actattctgg gcaaatcctt 1080
cgacagtaag ccatctctgt gccttgtgcc cctccccgtg gccctcgcgc gcagcatacc 1140
ctcccttgtg gtgcgactcc aacatgaagc tagcacgtgt aagcaaggca atcctcacct 1200
ctgttcacta attcgtgtcg ctctgcaact ccatactgat cagacaatgc cagagctggt 1260
ggtcaaccca acgaacggtc ttacgatcct gctgacctac cgcctcacta gtcctaccgg 1320
gagagttcga ctgacttgca gcagactgcc cacggaacct gtctgctcgc tgtagagatg 1380
gtgatgagtg atggtcgata tgccgacatc cttctctcgt tcgaccctag tgctctaccg 1440
atacggacgc ccttcgactc agccaaagga ggcatttgga cttggacaaa gcagaacata 1500
tcgcctctcg atcgatataa gtatcagcat ctcctcctgc cataattcaa ctttctcctc 1560
atcatccaac aaaccatcca atcctctaaa gcatctggct cacaacaagc ctctgctcat 1620
tcattcgctc taagtctttg aatcttcgat tcttcgatac ttctctcgct atgttctcca 1680
aggtccttcc cttcgtggga gcggttgccg ccctccctca ctccgtccgt caggagcctg 1740
gctccggcat tggctaccca tacgacaaca acaccctgcc atatgtcgcc ccaggtccta 1800
ccgactctcg tgctccttgc ccagctctga acgctcttgc caaccacggt tacattcctc 1860
acgatggccg tgccatcagc agggagaccc tccagaacgc tttcctcaac cacatgggta 1920
ttgccaactc cgtcattgag cttgctctga ccaacgcctt cgtcgtctgc gagtacgtta 1980
ctggctccga ctgtggtgac agccttgtca acctgactct gctcgccgag ccccacgctt 2040
tcgagcacga ccactccttc tcccgcaagg attacaagca gggtgtcgcc aactccaacg 2100
acttcatcga caacaggaac ttcgatgccg agaccttcca gacctctctg gatgtcgttg 2160
caggcaagac ccacttcgac tatgccgaca tgaacgagat ccgccttcag cgcgagtccc 2220
tctccaacga gcttgacttc cccggttggt tcaccgagtc caagccaatc cagaacgtcg 2280
agtctggctt catcttcgcc cttgtctctg acttcaacct gcccgacaac gatgagaacc 2340
ctctggttcg cattgactgg tggaagtact ggttcaccaa cgagtccttc ccataccacc 2400
tcggctggca ccccccgtct ccagccaggg agatcgagtt cgtcacctcc gcctcctccg 2460
ctgtcctggc tgcctctgtc acctctactc catcttccct tccatccggt gccatcggcc 2520
caggtgccga ggctgtccct ctctccttcg cctccaccat gaccccattc ctcctcgcca 2580
ccaatgctcc ttactacgcc caggacccaa ctctcggccc caacgacaag cgtgaggctg 2640
ccccagctgc caccacctcc atggccgtct tcaagaaccc atacctcgag gccattggca 2700
cccaggacat caagaaccag caggcttacg tcagctccaa ggctgctgcc atggcctctg 2760
ccatggccgc caacaaggcc cgcaaccttt aagcgcatct acgacatcga gatggtccag 2820
cattcactgt tccgaatgtg aatgtgctac ttaacgacaa cacgaggaaa tgcctttcct 2880

cactacagca tcagcatgca gaggcgcatt tggtttttcc cgcatagatc ggctggcttg 2940
gtcacgagtt ttccgattgc acgattgatt ttatagaata gaacgataga ctatagacat 3000
agaaaaaact gtatgcattt catgccgcgt tttaccccaa aacctatctc ctctcttttc 3060
gccgtctatt ttctcaattc cctctttcgt ttcttcccct ttggttttac agacaatcaa 3120
tcaatcgatc gatcgaaagg ctcattggcc ttcttacatt tactgttatc ccttccgcat 3180
tggtacagaa cagtgtggag gaacgcggta tgagatcata accatcatta caacatcatg 3240
gcagcgcaga tcgctgcgag cttcttaccc aggaaaggat cggggtatca ttaagctacc 3300
gccctggcct agccagtcgc ccgaagatct agcttgcagc ggctgcccta ttttctcacc 3360
catgcgggcc atgactgttc gcgaagttag tcccctatcc aagttgaaaa gatgggaaag 3420
tggagatttc ttacgtcgtg ttgactactt ttgccacttc ccttatcaga cgtatatact 3480
ccaacatctg atctgagtgt tagtataagg catctagaga cagaagatga tattgaagga 3540
gcactttttg ggcttggctg gagctagtgg aggtcaacaa tgaatgccta ttttggttta 3600
gtcgtccagg cggtgagcac aaaatttgtg tcgtttgaca agatggttca tttaggcaac 3660
tggtcagatc agccccactt gtagcagtag cggcggcgct cgaagtgtga ctcttattag 3720
cagacaggaa cgaggacatt attatcatct gctgcttggt gcacgataac ttggtgcgtt 3780
tgtcaagcaa ggtaagtgaa cgacccggtc ataccttctt aagttcgccc ttcctccctt 3840
tatttcagat tcaatctgac ttacctattc tacccaagca tcgatatgaa aaagcctgaa 3900
ctcaccgcga cgtctgtcga gaagtttctg atcgaaaagt tcgacagcgt ctccgacctg 3960
atgcagctct cggagggcga agaatctcgt gctttcagct tcgatgtagg agggcgtgga 4020
tatgtcctgc gggtaaatag ctgcgccgat ggtttctaca aagatcgtta tgtttatcgg 4080
cactttgcat cggccgcgct cccgattccg gaagtgcttg acattgggga attcagcgag 4140
agcctgacct attgcatctc ccgccgtgca cagggtgtca cgttgcaaga cctgcctgaa 4200
accgaactgc ccgctgttct gcagccggtc gcggaggcca tggatgcgat cgctgcggcc 4260
gatcttagcc agacgagcgg gttcggccca ttcggaccgc aaggaatcgg tcaatacact 4320
acatggcgtg atttcatatg cgcgattgct gatccccatg tgtatcactg gcaaactgtg 4380
atggacgaca ccgtcagtgc gtccgtcgcg caggctctcg atgagctgat gctttgggcc 4440
gaggactgcc ccgaagtccg gcacctcgtg cacgcggatt tcggctccaa caatgtcctg 4500
acggacaatg gccgcataac agcggtcatt gactggagcg aggcgatgtt cggggattcc 4560
caatacgagg tcgccaacat cttcttctgg aggccgtggt tggcttgtat ggagcagcag 4620
acgcgctact tcgagcggag gcatccggag cttgcaggat cgccgcggct ccgggcgtat 4680
atgctccgca ttggtcttga ccaactctat cagagcttgg ttgacggcaa tttcgatgat 4740
gcagcttggg cgcagggtcg atgcgacgca atcgtccgat ccggagccgg gactgtcggg 4800
cgtacacaaa tcgcccgcag aagcgcggcc gtctggaccg atggctgtgt agaagtactc 4860
gccgatagtg gaaaccgacg ccccagcact cgtccgaggg caaaggaata gagtagatgc 4920

cgaccgggat ccacttaacg ttactgaaat catcaaacag cttgacgaat ctggatataa 4980

gatcgttggt gtcgatgtca gctccggagt tgagacaaat ggtgttcagg atctcgataa 5040

gatacgttca tttgtccaag cagcaaagag tgccttctag tgatttaata gctccatgtc 5100

aacaagaata aaacgcgttt tcgggtttac ctcttccaga tacagctcat ctgcaatgca 5160

ttaatgcatt gactgcaacc tagtaacgcc ttcaggctcc ggcgaagaga agaatagctt 5220

agcagagcta ttttcatttt cgggagacga gatcaagcag atcaacggtc gtcaagagac 5280

ctacgagact gaggaatccg ctcttggctc cacgcgacta tatatttgtc tctaattgta 5340

ctttgacatg ctcctcttct ttactctgat agcttgacta tgaaaattcc gtcaccagcc 5400

ctgggttcgc aaagataatt gcatgtttct tccttgaact ctcaagccta caggacacac 5460

attcatcgta ggtataaacc tcgaaatcat tcctactaag atggtataca atagtaacca 5520

tggttgccta gtgaatgctc cgtaacaccc aatacgccgg ccgaaacttt tttacaactc 5580

tcctatgagt cgtttaccca gaatgcacag gtacacttgt gcggccgcgg tattgccact 5640

tctcgacaca cacacacaca ccggccaccc tacaggccgt ctacacctcc tctctggagc 5700

gaggacaaca tgagacaatc acagatgggg gggaggaata gggaagcaac gaggctgcca 5760

gcggcggaag gcagaaggcg gtgcgtgccc tttcccaatc tggcggcggc agcgaggtgg 5820

gcgggcaatg tactgggagg catgggagcg gccacccaga tggctccaga gggcctggga 5880

ggggtgtgtg aggcgtgtgt gaccaagtca gtgagtgcgc gcagtttccc agcacccggc 5940

gatgcgcgcg cgcgcgtctc tcgcaggccc ggcaccaaaa cggtgtatgc gtgggacgca 6000

cttttccggg ggcggctcgc cgcacacgag gatcaaagtc catgacgttc ggaggatata 6060

taagaagaaa gcgtctcaca gtttcagaaa aagcgtaaaa accttttctc tggagcgatg 6120

tcccctgatg gaaggtcgta atgggctagc atctgggggc cctaggcgct ctctatggcg 6180

ggcctcctgg cctcgacttt gatgggttct ggggtgttgt agtcgcgggg caacccgttt 6240

gactcccccc aaaggtcccc cctgcaggat gctgcatgcg caggcacacg cttcgagtca 6300

ctcggcgagg tgttgcgccc cgaaactccc aaggaggtgg tc 6342

<210> 9
<211> 712
<212> DNA
<213> Caldariomyces fumago

<400> 9
tattgccact tctcgacaca cacacacaca ccggccaccc tacaggccgt ctacacctcc 60

tctctggagc gaggacaaca tgagacaatc acagatgggg gggaggaata gggaagcaac 120

gaggctgcca gcggcggaag gcagaaggcg gtgcgtgccc tttcccaatc tggcggcggc 180

agcgaggtgg gcgggcaatg tactgggagg catgggagcg gccacccaga tggctccaga 240

gggcctggga ggggtgtgtg aggcgtgtgt gaccaagtca gtgagtgcgc gcagtttccc 300

agcacccggc gatgcgcgcg cgcgcgtctc tcgcaggccc ggcaccaaaa cggtgtatgc 360

gtgggacgca cttttccggg ggcggctcgc cgcacacgag gatcaaagtc catgacgttc 420

ggaggatata taagaagaaa gcgtctcaca gtttcagaaa aagcgtaaaa accttttctc 480

tggagcgatg tcccctgatg gaaggtcgta atgggctagc atctgggggc cctaggcgct 540

ctctatggcg ggcctcctgg cctcgacttt gatgggttct ggggtgttgt agtcgcgggg 600

caacccgttt gactcccccc aaaggtcccc cctgcaggat gctgcatgcg caggcacacg 660

cttcgagtca ctcggcgagg tgttgcgccc cgaaactccc aaggaggtgg tc 712

<210> 10
<211> 723
<212> DNA
<213> Caldariomyces fumago

<400> 10
tcagttcaaa agcccatgcc tgctcccagt ctgggggcag tttgggtgtg gagggatcgg 60

ggcagggcca tccccggcgc gagatacact tgggctccac gacccttggg tgacagagct 120

gcgcagcagg tagacaggta gaccagagac cggagaccgg gcctgcctgc ccatgcggtc 180

cgccacaggg ggatttggag gaaggaacga agcccagggc gcaggtacag gtgtgtgccc 240

ctcgaagccg cgagggtggc cactggggca gagtaggctg gagagcgtgg gaggtgagac 300

tggtgccgca tggagctggg ccgcgacagc caccacctcc cacatggagg aagcgagctg 360

ctggcagcgg tgcgcgttgg ggtccgggca gggccacccc cacgtgccac agccccattg 420

cggcgttggg aggcggcgtt gggagatgag gcagggtcgc ccgggacggc gttgggctcg 480

gggcagggtc accccgtgag ggctgcagcc tacaggcggc ttgctctggt cccggacatt 540

ctgggacctc ttacctctga cctccgagct cttgcctctg ccctctggcg ttggcattct 600

ttggggcccg tccagcgctc cggccgcggc gttggcaggg gcctggcggc gttggcatca 660

gggctcagcc ctccagccgt ggcgttgggg ggcagcggac cggttgctca cttgccgtgt 720

atc 723

<210> 11
<211> 19
<212> DNA
<213> künstliche Sequenz

<400> 11
gtagtcatat gcttgtctc 19

<210> 12
<211> 21
<212> DNA
<213> künstliche Sequenz

<400> 12
ggctgctggc accagacttg c 21

<210> 13
<211> 17
<212> DNA
<213> künstliche Sequenz

<400>  13
gaccctgttg agcttga                                                    17


<210>  14
<211>  17
<212>  DNA
<213>  künstliche Sequenz

<400>  14
gacttagagg cgttcag                                                    17


<210>  15
<211>  22
<212>  DNA
<213>  künstliche Sequenz

<400>  15
agtgccgcga agctaccatc tg                                              22


<210>  16
<211>  26
<212>  DNA
<213>  künstliche Sequenz

<400>  16
ttaccacggt tatccatgta gtaagg                                          26


<210>  17
<211>  33
<212>  DNA
<213>  künstliche Sequenz

<400>  17
tgcagcggcc gcggtattgc cacttctcga cac                                  33


<210>  18
<211>  31
<212>  DNA
<213>  künstliche Sequenz

<400>  18
tgcagagctc gaccacctcc ttgggagttt c                                    31


<210>  19
<211>  32
<212>  DNA
<213>  künstliche Sequenz

<400>  19
tgcaggtacc tcagttcaaa agcccatgcc tg                                   32


<210>  20
<211>  31
<212>  DNA
<213>  künstliche Sequenz

<400>  20
tgcagtcgac gatacacggc aagtgagcaa c                                    31


<210>  21

<211>  38
<212>  DNA
<213>  künstliche Sequenz

<400>  21
actgaagctt agatctctag agacagaaga tgatattg                              38


<210>  22
<211>  34
<212>  DNA
<213>  künstliche Sequenz

<400>  22
agctgcggcc gcacaagtgt acctgtgcat tctg                                  34


<210>  23
<211>  32
<212>  DNA
<213>  künstliche Sequenz

<400>  23
actgaagctt ctggtttgca tgcacttgca tc                                    32


<210>  24
<211>  35
<212>  DNA
<213>  künstliche Sequenz

<400>  24
actgtctaga tgccttatac taacactcag atcag                                 35


<210>  25
<211>  22
<212>  DNA
<213>  künstliche Sequenz

<400>  25
agcccgttgt cggctcgatt tg                                               22


<210>  26
<211>  22
<212>  DNA
<213>  künstliche Sequenz

<400>  26
aagcttatcg ataccgtcga cg                                               22


<210>  27
<211>  22
<212>  DNA
<213>  künstliche Sequenz

<400>  27
tcgtttaccc agaatgcaca gg                                               22


<210>  28
<211>  21
<212>  DNA
<213>  künstliche Sequenz

<400>  28

aaccaccaag gcgtctacct g 21